# EUROPEAN PATENT SPECIFICATION

(11) **EP 3 637 422 B1**
(45) Date of publication and mention of the grant of the patent: **20.12.2023**
(21) Application number: 19199379.9
(22) Date of filing: 14.03.2014
(51) Int. Cl.: G16B 20/20, G16B 30/00

(54) **COMPUTER FILES AND METHODS SUPPORTING FORENSIC ANALYSIS OF NUCLEOTIDE SEQUENCE DATA**
COMPUTERDATEIEN UND VERFAHREN ZUR UNTERSTÜTZUNG DER FORENSISCHEN ANALYSE VON NUKLEOTIDSEQUENZDATEN
FICHIERS INFORMATIQUES ET PROCÉDÉS PRENANT EN CHARGE L'ANALYSE MÉDICO-LÉGALE DE DONNÉES DE SÉQUENCE DES NUCLÉOTIDES

(30) Priority: 15.03.2013 US 201313834830; 29.07.2013 US 201313952761
(43) Date of publication of application: 15.04.2020
(62) Divisional of application: 14716722.5
(73) Proprietor: Battelle Memorial Institute, Columbus, OH 43201-2969 (US)
(72) Inventor: YOUNG, Brian A., Columbus, Ohio 43235 (US); MINARD-SMITH, Angela T., Marysville, Ohio 43040 (US); HEIZER, Esley M. Jr., Beavercreek, Ohio 45440 (US); BORNMAN, Daniel M., Powell, Ohio 43065 (US); HESTER, Mark E., Reynoldsburg, Ohio 43068 (US); YANG, Boyu, Charlottesville, Virginia 22911 (US)
(74) Representative: HGF

(56) References cited:
- RASMUS NIELSEN ET AL: "Genotype and SNP calling from next-generation sequencing data", NATURE REVIEWS GENETICS, vol. 12, no. 6, 1 June 2011 (2011-06-01), pages 443-451, XP055046801, ISSN: 1471-0056, DOI: 10.1038/nrg2986
- MING CHEN ET AL: "MfSAT: Detect simple sequence repeats in viral genomes", BIOINFORMATION, vol. 6, no. 4, 1 January 2011 (2011-01-01) , pages 171-172, XP055126243, ISSN: 0973-8894, DOI: 10.6026/97320630006171
- BIZZARO JEFF W ET AL: "Poly: a quantitative analysis tool for simple sequence repeat (SSR) tracts in DNA", BMC BIOINFORMATICS, BIOMED CENTRAL, LONDON, GB, vol. 4, no. 1, 5 June 2003 (2003-06-05), page 22, XP021013590, ISSN: 1471-2105, DOI: 10.1186/1471-2105-4-22
- Anonymous: "Microsatellite - Wikipedia, the free encyclopedia", , 28 February 2013 (2013-02-28), XP055126318, Retrieved from the Internet: URL:http://en.wikipedia.org/w/index.php?ti tle=Microsatellite&oldid=541184326 [retrieved on 2014-07-02]

## Description

### SEQUENCE LISTING

This application contains a Sequence Listing which has been submitted in ASCII format via EFS-Web and is hereby incorporated by reference in its entirety. Said ASCII copy, created on March 12, 2014, is named 920006-228998_SL.txt and is 18,452 bytes in size.

### TECHNICAL FIELD

The present disclosure relates, generally, to nucleotide sequence data and, more particularly, to computer files and methods supporting forensic analysis of nucleotide sequence data.

### BACKGROUND ART

Polymorphic tandem repeats of nucleotide sequences are found throughout the human genome, and the particular combinations of alleles identified by their multiple repeat sites are sufficiently unique to an individual that these repeating sequences can be used in human or other organism identification. These markers are also useful in genetic mapping and linkage analysis, where the tandem repeat sites may be useful for determining, for example, predisposition for disease. Tandem repeats can be used directly in human identity testing, such as in forensics analysis. There are many types of tandem repeats of nucleic acids, falling under the general term variable number tandem repeats (VNTR). For example, mini satellites and microsatellites are VNTRs, and microsatellites include short tandem repeats (STRs).

One application of tandem repeat analysis is in forensics or human identity testing. In current forensics analyses, highly polymorphic STRs are identified using a deoxyribonucleic acid (DNA) sample from an individual and DNA amplification steps, such as polymerase chain reaction (PCR), to provide amplified samples of partial DNA sequences, or amplicons, from the individual's DNA. The amplicons can then be matched by size (i.e., repeat numbers) to reference databases, such as the sequences stored in national or local DNA databases. For example, amplicons that originate from STR loci can be matched to reference STR databases, including the Federal Bureau of Investigation (FBI) Combined DNA Index System (CODIS) database in the United States, or the National DNA Database (NDNAD) in the United Kingdom, to identify the individual by matching to the STR alleles specific to that individual.

Forensic DNA analysis is about to cross a threshold where DNA samples will begin to be analyzed routinely by massively parallel sequencing (MPS), also sometimes referred to in the art as next-generation sequencing. The advent of routine MPS for forensic DNA analysis will create large quantities of nucleotide sequence data that may enable richer exploitation of DNA in forensic applications. Once information is generated on the genetic profile of an individual (e.g., for either forensic investigative purposes or confirmatory matching), the resulting nucleotide sequence data should be formatted for exchange among law enforcement entities. Moreover, forensic analysis requires the preservation of data, including raw data, for evidentiary purposes. Data files created by MPS workflows are typically larger than 1 GB, making them difficult to transmit or store. In addition, these files, while text-based, are not human-readable in any practical sense because of their large size. Thus, other human readable forms of the data from MPS workflows are needed.

RASMUS NIELSEN ET AL, "Genotype and SNP calling from next-generation sequencing data", NATURE REVIEWS GENETICS, (20110601), vol. 12, no. 6, doi:10.1038/nrg2986, ISSN 1471-0056, pages 443 - 451, appears to disclose a review of statistical methods of genotype calling and to provide a guide for the use of these methods in next-generation sequencing (NGS) studies.

### DISCLOSURE OF INVENTION

According to the invention, an allelotyping method may comprise selecting, from among a group of text strings that each represent a nucleotide sequence that was read by a massively parallel sequencing (MPS) instrument, a plurality of text strings for which the nucleotide sequences represented by the selected plurality of text strings each correspond to a particular locus; comparing the selected plurality of text strings to one another to determine an abundance count for each unique text string included in the selected plurality of text strings; and determining one or more alleles for the particular locus by comparing the abundance count for each unique text string included in the selected plurality of text strings to an abundance threshold.

Further embodiments of the invention are defined in the dependent claims of the appended claim set.

According to one aspect of the disclosure, a method may comprise receiving a first text-based computer file including one or more records, each of the one or more records comprising nucleotide sequence data generated by a read of a massively parallel sequencing (MPS) instrument, determining, for each of the one or more records of the first text-based file, whether a portion of the nucleotide sequence data of the record represents a short tandem repeat (STR) associated with a locus, placing each portion of the nucleotide sequence data determined to represent an STR associated with a locus into one of a number of locus-specific lists, determining, for each of the locus-specific lists, a number of occurrences within the locus-specific list of identical nucleotide sequence data representing a unique STR, and generating a second text-based computer file including one or more records, each of the one or more records corresponding to a unique STR for which the number of occurrences of identical nucleotide sequence data representing the unique STR exceeded an abundance threshold.

In some examples, the first text-based computer file may be formatted as a FASTQ file. Determining whether a portion of the nucleotide sequence data of a record represents an STR associated with a locus may comprise determining whether a portion of the nucleotide sequence data of the record represents a primer sequence used to amplify the locus. Determining whether a portion of the nucleotide sequence data of the record represents a primer sequence used to amplify the locus may comprise referencing an updateable library of primer sequences. Placing each portion of the nucleotide sequence data determined to represent an STR associated with a locus into one of a number of locus-specific lists may comprise removing a portion of the nucleotide sequence data representing a flanking sequence. Removing a portion of the nucleotide sequence data representing a flanking sequence may comprise referencing an updateable library of flanking sequences. The abundance threshold may be user-defined.

In some examples, generating the second text-based computer file may comprise, for each of the one or more records, writing nucleotide sequence data representing the corresponding unique STR to a second text line of the record. Generating the second text-based computer file may further comprise, for each of the one or more records, writing average quality scores for the nucleotide sequence data representing the corresponding unique STR to a fourth text line of the record. The average quality scores may be formatted as average Phred quality scores. Generating the second text-based computer file may further comprise, for each of the one or more records, writing forensic metadata associated with the nucleotide sequence data representing the corresponding unique STR to a first text line of the record. The forensic metadata may be copied from the first text-based computer file. Generating the second text-based computer file may further comprise, for each of the one or more records, writing an attribute-value pair specifying the number of occurrences of identical nucleotide sequence data representing the corresponding unique STR to a third text line of the record.

In some examples, generating the second text-based computer file may further comprise, for each of the one or more records, writing a human-readable sequence-based allele (HRSBA) designation that is deterministic of the corresponding unique STR to a third text line of the record. Generating the HRSBA designation may comprise reading a plurality of nucleotide bases in a sliding window that moves along the nucleotide sequence data, determining whether the plurality of nucleotide bases corresponds to a canonical motif of a locus associated with the corresponding unique STR, adding the plurality of nucleotide bases to the HRSBA in response to determining that the plurality of nucleotide bases corresponds to a canonical motif of the locus and represents a first instance of the canonical motif, and moving the sliding window by a plurality of positions in response to determining that the plurality of nucleotide bases corresponds to a canonical motif of the locus.

In some examples, generating the HRSBA designation may further comprise adding only a first nucleotide base of the plurality of nucleotide bases to the HRSBA in response to determining that the plurality of nucleotide bases does not correspond to a canonical motif of the locus and moving the sliding window by one position in response to determining that the plurality of nucleotide bases does not correspond to a canonical motif of the locus. The sliding window may move along the nucleotide sequence data in a 5' to 3' direction. Determining whether the plurality of nucleotide bases corresponds to a canonical motif of a locus associated with the corresponding unique STR may comprise referencing an updatable library of canonical motifs of one or more loci. Generating the HRSBA designation may further comprise determining whether a final plurality of nucleotide bases of the nucleotide sequence corresponds to a canonical ending motif of a locus associated with the corresponding unique STR and generating a user alert in response to determining that the final plurality of nucleotide bases does not correspond to a canonical ending motif of the locus. Generating the HRSBA designation may comprise referencing an updatable library associating common nucleotide sequences with corresponding HRSBA designations.

According to another aspect of the disclosure, an allelotyping method may comprise selecting a plurality of text strings that each represent a nucleotide sequence that was read by a massively parallel sequencing (MPS) instrument, where the nucleotide sequences represented by the selected plurality of text strings each correspond to a particular locus, comparing the selected plurality of text strings to one another to determine an abundance count for each unique text string included in the selected plurality of text strings, and determining one or more alleles for the particular locus by comparing the abundance count for each unique text string included in the selected plurality of text strings to an abundance threshold.

In some examples, determining the one or more alleles for the particular locus may comprise identifying one or more unique text strings that each represent a nucleotide sequence containing a short tandem repeat (STR). In other examples, determining the one or more alleles for the particular locus may comprise identifying one or more unique text strings that each represent a nucleotide sequence containing a single nucleotide polymorphism (SNP).

In some examples, comparing the abundance count for each unique text string included in the selected plurality of text strings to the abundance threshold may comprise identifying the unique text string having a highest abundance count from among the selected plurality of text strings. Comparing the abundance count for each unique text string included in the selected plurality of text strings to the abundance threshold may further comprise calculating whether a ratio of the abundance count for each unique text string compared to the highest abundance count exceeds the abundance threshold. The abundance threshold may be a percentage value in the range of 15% to 60%. The abundance threshold may be a percentage value that is configurable by a user.

In some examples, the allelotyping method may further comprise receiving a first text-based computer file comprising a plurality of text strings that each represent a nucleotide sequence that was read by the MPS instrument, prior to selecting the plurality of text strings for which the represented nucleotide sequences each correspond to the particular locus, and generating a second text-based computer file comprising each unique text string for which the ratio exceeds the abundance threshold, where a file size of the second text-based computer file is smaller than a file size of the first text-based computer file. The second text-based computer file may comprise one or more unique text strings that each represent a nucleotide sequence determined to be an allele for the particular locus. The file size of the second text-based computer file may be at least ten-thousand times smaller than the file size of the first text-based computer file.

In some examples, the steps of (i) selecting the plurality of text strings for which the represented nucleotide sequences each correspond to a particular locus, (ii) comparing the selected plurality of text strings to one another to determine the abundance count for each unique text string included in the selected plurality of text strings, and (iii) determining one or more alleles for the particular locus by comparing the abundance counts to the abundance threshold may be performed for each of a plurality of loci present in a sample that was read by the MPS instrument. Selecting the plurality of text strings for which the represented nucleotide sequences each correspond to one of the plurality of loci may comprise determining whether each of a plurality of text strings generated by the MPS instrument when reading the sample includes text characters that represent a flanking nucleotide sequence associated with a particular locus and selecting a plurality of text strings that include the text characters that represent the flanking nucleotide sequence associated with the particular locus.

In some examples, the allelotyping method may further comprise removing the text characters that represent the flanking nucleotide sequence from each of the selected plurality of text strings prior to comparing the selected plurality of text strings to one another to determine the abundance count for each unique text string included in the selected plurality of text strings. The allelotyping method may further comprise removing all text characters that do not represent a short tandem repeat (STR) from each of the selected plurality of text strings prior to comparing the selected plurality of text strings to one another to determine the abundance count for each unique text string included in the selected plurality of text strings.

According to yet another aspect of the disclosure, a method may comprise receiving forensic metadata associated with nucleotide sequence data generated by a massively parallel sequencing (MPS) instrument and writing the forensic metadata to a text-based computer file comprising the nucleotide sequence data. In some examples, receiving the forensic metadata may comprise receiving data from a case management system of a laboratory operating the MPS instrument. The text-based computer file comprising the nucleotide sequence data may include one or more records, each of the one or more records representing a read of the MPS instrument. The text-based computer file comprising the nucleotide sequence data may be formatted as a FASTQ file.

In some examples, writing the forensic metadata to the text-based computer file may comprise writing the forensic metadata to a first text line of each of the one or more records of the text-based computer file. The first text line of each of the one or more records of the text-based computer file may comprise a unique sequence identifier created by the MPS instrument when generating the nucleotide sequence data. Writing the forensic metadata to the first text line of each of the one or more records of the text-based computer file may comprise appending the forensic metadata to the unique sequence identifier. Writing the forensic metadata to the text-based computer file may comprise writing one or more attribute-value pairs to the text-based computer file, each of the one or more attribute-value pairs specifying one of a file format, a unique case identifier, a unique sample identifier, a unique laboratory identifier, and a unique technician identifier.

According to still another aspect of the disclosure, a computer-readable medium may store a text-based file comprising one or more records. Each of the one or more records may include a first text line comprising forensic metadata, a second text line comprising a number of characters representing nucleotide sequence data, a third text line, and a fourth text line comprising a number of characters representing quality scores associated with the nucleotide sequence data.

In some examples, each of the characters of the second text line may represent an output of a base call algorithm performed by a massively parallel sequencing (MPS) instrument. The first text line may further comprise a unique sequence identifier created by a massively parallel sequencing (MPS) instrument when generating the nucleotide sequence data. The forensic metadata of the first text line may comprise one or more attribute-value pairs, each of the one or more attribute-value pairs specifying one of a file format, a unique case identifier, a unique sample identifier, a unique laboratory identifier, and a unique technician identifier.

In some examples, the nucleotide sequence data may represent a short tandem repeat (STR) for which a read count from a sample exceeded an abundance threshold. The third text line may comprise an attribute-value pair specifying the read count of the STR. Each of the characters of the fourth text line may represent an average quality score associated with a corresponding character of the second text line, the average quality score being a function of quality scores associated with all reads of the STR. Each of the characters of the fourth text line may be formatted as an average Phred quality score. The third text line may comprise one or more attribute-value pairs, each of the one or more attribute-value pairs specifying one of a locus of the STR, a strand of the STR, an analytic threshold associated with the STR, and a designation of the STR as corresponding to one of an allele, a stutter, and an artifact.

In some examples, the third text line may comprise a human-readable sequence-based allele (HRSBA) designation that is deterministic of the STR. The HRSBA designation may comprise a first attribute-value pair summarizing the STR in a 5' to 3' direction. The first attribute-value pair of the HRSBA designation may comprise one or more integers each followed by one or more characters, each of the one or more integers representing a nucleotide position and each of the one or more characters representing a nucleotide base. The one or more characters following each of the one or more integers in the first attribute-value pair of the HRSBA designation may be bracketed if the one or more characters correspond to a repeat motif of the STR. Each of the characters in the first attribute-value pair of the HRSBA designation may be an International Union of Pure and Applied Chemistry (ILTPAC) nucleotide base code. The HRSBA designation may comprise a second attribute-value pair specifying a length of the STR.

According to still yet another aspect of the disclosure, a computer-readable medium may store a text-based computer file comprising one or more records. Each of the one or more records may include a first text line, a second text line comprising a text string representing a nucleotide sequence containing a single nucleotide polymorphism (SNP), a third text line comprising a human-readable allele designation for the SNP, and a fourth text line.

In some examples, the human-readable allele designation of the third text line may comprise a number of attribute-value pairs that specify a first SNP state, a second SNP state, an abundance count of the first SNP state, an abundance count of the second SNP state, and a strand of the nucleotide sequence represented in the second text line. The human-readable allele designation of the third text line may further comprise an attribute-value pair specifying a reference SNP identifier associated with the nucleotide sequence represented in the second text line.

In some examples, the first text line may comprise a unique sequence identifier created by a massively parallel sequencing (MPS) instrument when generating data related to the nucleotide sequence represented in the second text line. The first text line may further comprise forensic metadata specifying one or more of a file format, a unique case identifier, a unique sample identifier, a unique laboratory identifier, and a unique technician identifier. The fourth text line may comprise a text string representing quality scores associated with the nucleotide sequence represented in the second text line.

### BRIEF DESCRIPTION OF DRAWINGS

The concepts described in the present disclosure are illustrated by way of example and not by way of limitation in the accompanying figures. For simplicity and clarity of illustration, elements illustrated in the figures are not necessarily drawn to scale. For example, the dimensions of some elements may be exaggerated relative to other elements for clarity. Further, where considered appropriate, reference labels have been repeated among the figures to indicate corresponding or analogous elements. The detailed description particularly refers to the accompanying figures in which:
FIG. 1 is a simplified flow diagram illustrating one embodiment of a method of creating a computer file comprising raw nucleotide sequence data and forensic metadata;
FIG. 2 illustrates a portion of one embodiment of a text-based computer file comprising raw nucleotide sequence data (SEQ ID NOS 4-6, respectively, in order of appearance);
FIG. 3 illustrates a portion of one embodiment of a text-based computer file comprising raw nucleotide sequence data (SEQ ID NOS 7-9, respectively, in order of appearance) and forensic metadata;
FIG. 4 is a simplified flow diagram illustrating one embodiment of a method of creating a computer file comprising summary nucleotide sequence data and metadata;
FIG. 5 illustrates a portion of one embodiment of a text-based computer file comprising summary nucleotide sequence data (SEQ ID NOS 10-13, respectively, in order of appearance) and metadata;
FIG. 6 illustrates pseudo-code for a motif aware allellotyper (MAA) algorithm;
FIG. 7 graphically illustrates one example of the operation of the MAA algorithm of FIG. 6 (FIG. 7 discloses the top sequence as SEQ ID NO: 14, "1[AGAT]5... 17[AGAT]21" as SEQ ID NO: 14, and "1[AGAT]5... 1[AGAT]" as SEQ ID NO: 14);
FIG. 8 graphically illustrates another example of the operation of the MAA algorithm of FIG. 6 (FIG. 8 discloses the sequences in the middle column as SEQ ID NOS 15 and 15 and the sequences in the right column, beginning with "1[TCTA]13," as SEQ ID NOS 16-44 and 15, respectively, in order of appearance);
FIG. 9 illustrates a portion of one embodiment of a look-up table that may be contained in an updatable library (FIG. 9(d) discloses SEQ ID NOS 45, 45-46, 46-47, 47-48, 48-49, 49-50, 50-51, 51-52, 52-53, 53-54 and 54, respectively, in order of appearance);
FIG. 10 is a simplified flow diagram illustrating one embodiment of an allelotyping method;
FIG. 11 shows portions of an illustrative table including unique text strings that each represent a nucleotide sequence (SEQ ID NOS 2, 3, and 55-72, respectively, in order of appearance) corresponding to a particular locus, in which the unique text strings have been sorted by abundance count using the allelotyping method of FIG. 10;
FIG. 12 is a graph of the abundance counts of the top ten unique text strings sorted by abundance from FIG. 11; and
FIG. 13 illustrates a portion of one embodiment of an SEF file comprising human-readable allele designations for several single nucleotide polymorphisms (SNPs) (SEQ ID NOS 73-75, respectively, in order of appearance).

### BEST MODE(S) FOR CARRYING OUT THE INVENTION

While the concepts of the present disclosure are susceptible to various modifications and alternative forms, specific embodiments thereof have been shown by way of example in the drawings and will be described herein in detail. It should be understood, however, that there is no intent to limit the concepts of the present disclosure to the particular forms disclosed, but on the contrary, the intention is to cover all modifications, equivalents, and alternatives consistent with the present disclosure and the appended claims.

References in the specification to "one embodiment," "an embodiment," "an illustrative embodiment," etc., indicate that the embodiment described may include a particular feature, structure, or characteristic, but every embodiment may or may not necessarily include that particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to effect such feature, structure, or characteristic in connection with other embodiments whether or not explicitly described.

The disclosed embodiments may be implemented, in some cases, in hardware, firmware, software, or any combination thereof. The disclosed embodiments may also be implemented as data and/or instructions carried by or stored on a transitory or non-transitory computer-readable storage medium, which may be read and executed by one or more processors. A computer-readable storage medium may be embodied as any storage device, mechanism, or other physical structure for storing or transmitting information in a form readable by a computing device (e.g., a volatile or non-volatile memory, a media disc, or other media device).

In the drawings, some structural or method features may be shown in specific arrangements and/or orderings. However, it should be appreciated that such specific arrangements and/or orderings may not be required. Rather, in some embodiments, such features may be arranged in a different manner and/or order than shown in the illustrative figures. Additionally, the inclusion of a structural or method feature in a particular figure is not meant to imply that such feature is required in all embodiments and, in some embodiments, may not be included or may be combined with other features.

Among other aspects, the present disclosure relates to a new computer file format useful for storing, exchanging, and/or preserving evidence regarding nucleotide sequence data, including short tandem repeat (STR) and/or single nucleotide polymorphism (SNP) profiling data, particularly when the data originates from a massively parallel sequencing (MPS) instrument or workflow. As used herein, the term "nucleotide sequence data" generally refers to symbolic representations of nucleotides arranged in sequential fashion. This new computer file format, which is illustratively referred to herein as the "Sequence Evidence Format" (SEF), accommodates the conventions and requirements of MPS-derived data, as well as the conventions and requirements of evidence preservation in forensic DNA analysis. For example, MPS-derived data typically conforms to the following conventions, among others: (1) use of text-based computer files for data exchange, (2) storage of the raw nucleotide sequence data, (3) storage of per-nucleotide quality scores in Phred format, (4) storage of sequencer-derived metadata, and (5) traceability of data to the level of individual reads. Forensic DNA analysis may require (1) recording of information necessary for evidence traceability, including case management metadata, (2) expression of STR alleles in a human-readable format, and (3) translation of allele lengths into a CODIS-compatible format. In at least some examples, the SEF addresses each of the foregoing concerns.

The SEF achieves compatibility with MPS workflows by incorporating certain aspects of the FASTQ file format that is output by many MPS instruments (and, thus, currently serves as a de facto industry standard). First, the SEF uses a text-based format that allows human readability. Second, the SEF uses a repeating four-row record convention. Unlike FASTQ, however, the SEF supports the evidentiary requirements inherent to forensic DNA analysis through the addition of certain metadata content.

In some illustrative examples, an SEF file may conform to the file format specification outlined in Table 1 and further described below. Table 1 sets forth an SEF specification with reference to the FASTQ file (the third column denoting several distinguishing features of SEF files, as compared to FASTQ files). It will be appreciated that, in other examples, an SEF file may be formatted according to additional or different specifications than those set forth in Table 1.

| Table 1 | | |
|---|---|---|
| Line Type | FASTQ | SEF (Differences from FASTQ) |
| 1) Title and Description | First character must be "@" | • The systematic identifier produced by MPS instrument/software may comprise the initial portion of this field |
| | • Free format field with no length limitation | |
| | • Arbitrary content can be included | • Forensic metadata as a series of optional attribute-value pairs |
| 2) Sequence | • No specific initial character is required for this line type | • Only International Union of Pure and Applied Chemistry (ILTPAC) nucleotide base codes are permitted ("ACTGNURYSWKMBDHVN.-") |
| | • Any printable character is permitted | |
| | • Upper case, lower case, and mixed case accepted | |
| | • Can be line wrapped | |
| 3) End of Sequence | • First character must be "+" | • Content after "+" does not have to match the title line |
| | • If the title line is repeated, it must be identical | |
| | | • STR metadata as a series of optional attribute-value pairs |
| 4) Quality Scores | • Accepts printable ASCII characters 33-126 | |
| | • Can be line wrapped | |
| | • Length must be equal to sequence length | |

Similar to FASTQ files, an SEF file may comprise one or more records, where each record includes data in four text lines. The first and third text lines of a record may contain metadata related to case management and to evidence preservation. The second text line contains nucleotide sequence data, while the fourth text line may contain quality scores for the nucleotide calls represented by the second text line. In the illustrative example, the quality scores of the fourth text line are formatted as Phred quality scores. SEF files may contain either raw nucleotide sequence data or summary nucleotide sequence data and metadata (e.g., created by an allelotyping process). As described further below, an attribute-value pair within the first text line of each record may indicate whether a particular file contains raw or summary data and metadata.

The present disclosure also relates to methods of creating and/or processing SEF files. For instance, a pair of algorithms (illustratively referred to in the present disclosure as "SEF Creator Raw" and as "SEF Creator Summary," respectively) may be used to create SEF files containing raw or summary data and metadata. The SEF Creator Raw and SEF Creator Summary algorithms are designed for use at different stages of the forensic DNA analysis process. In some examples, these two SEF file creation algorithms may be complemented by a number of utility algorithms that convert SEF files to other formats and otherwise manipulate SEF files (illustratively referred to herein as "SEFtools").

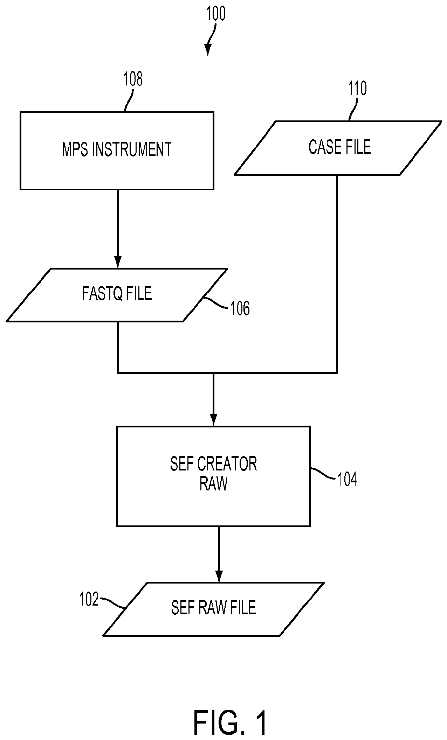

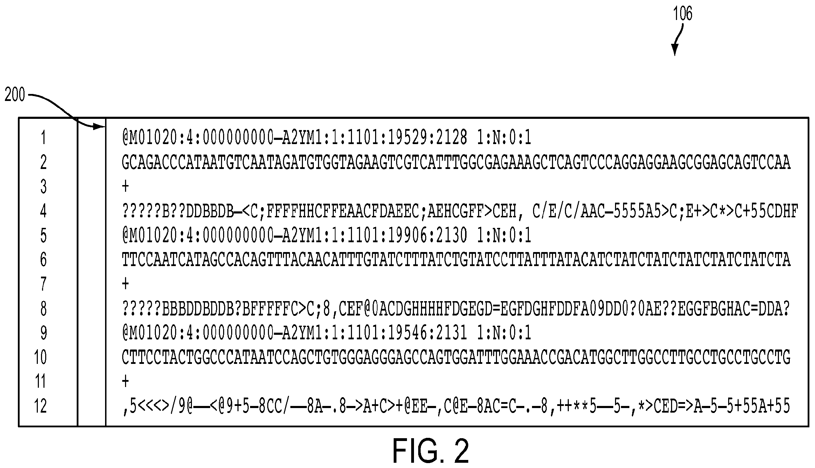

Referring now to FIG. 1, a method 100 of creating SEF files containing raw nucleotide sequence data and forensic metadata is shown as a simplified flow diagram. The method 100 is intended for use near the beginning of the forensic DNA analysis process to create an SEF file 102 that may serve as an evidentiary record comprising both raw nucleotide sequence data and case management information. As shown in FIG. 1, the SEF Creator Raw algorithm 104 may ingest data from two different sources, among others. Raw nucleotide sequence data, quality scores associated with the nucleotide sequence data, and instrument-specific metadata may be ingested from a FASTQ file 106 generated by an MPS instrument 108 (and its associated software). The first twelve lines (i.e., three records) of one illustrative example of a FASTQ file 106, output from an Illumina MiSeq MPS instrument, are shown in FIG. 2. As shown in FIG. 2, a first text line of each record of the FASTQ file 106 includes a unique sequence identifier 200 created by the MPS instrument 108, a second text line of each record of the FASTQ file 106 comprises a number of characters representing nucleotide sequence data, and a fourth text line of each record of the FASTQ file 106 comprises a number of characters representing quality scores associated with the nucleotide sequence data. Each record of the FASTQ file 106 represents a read of the MPS instrument 108, and each of the characters of the second text line represents an output of a base call algorithm performed by the MPS instrument 108.

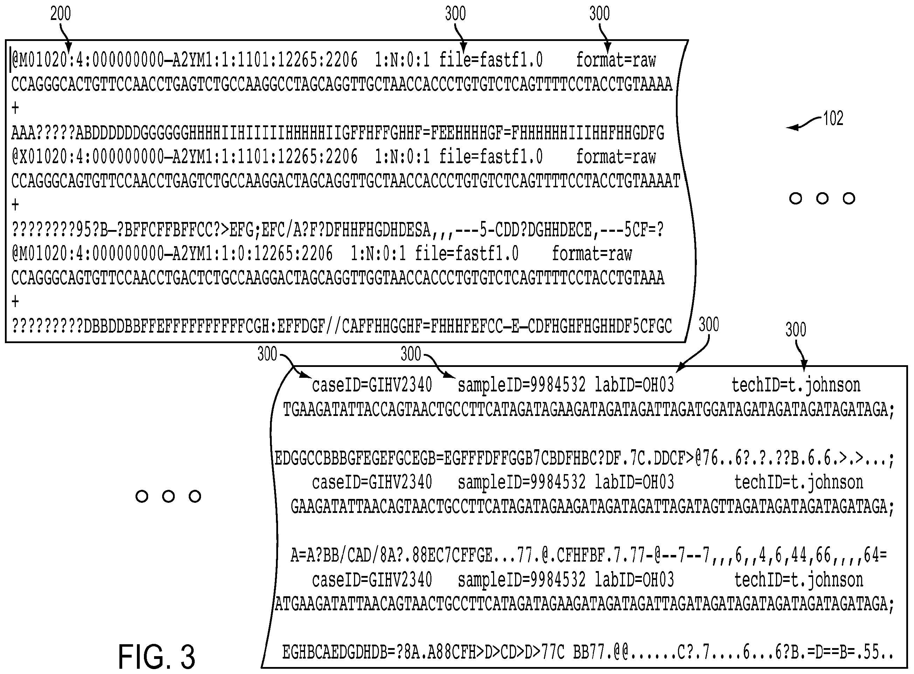

The SEF Creator Raw algorithm 104 may also ingest a case file 110 received from a laboratory case management system. In other examples of the method 100, the case management information may be manually input by a user of the MPS instrument via a user interface. Using these inputs, the algorithm 104 may write forensic metadata to each record of contained in the FASTQ file 106 a text-based computer file comprising the nucleotide sequence data. The output of the algorithm 104 is an SEF raw-format file 102, one illustrative example of which is shown in FIG. 3 (showing the first twelve lines, i.e. three records, of the file 102). In some examples, the SEF raw-format file 102 may include all of the data contained in the FASTQ file 106, plus the newly written forensic metadata. It is contemplated that, in other examples, the SEF raw-format file 102 need not include all of the data from the FASTQ file 106.

As mentioned above, the SEF raw-format file 102 includes forensic metadata (e.g., derived from the case file 110) in a first text line of each record of the file 102. In some examples, the forensic metadata may be in the form of one or more attribute-value pairs 300. In the illustrative example of FIG. 3, the first text line of each record contains several attribute-value pairs 300, with each pair tab-separated. In some examples, the attribute-value pairs 300 may specify one or more of a file format, a unique case identifier, a unique sample identifier, a unique laboratory identifier, and a unique technician identifier associated with the file 102. Several illustrative attribute-value pairs 300 are set forth in Table 2 below, including possible permissible values and intended purposes.

| Table 2 | | |
|---|---|---|
| Attribute | Permissible Values | Purpose |
| fileX.Y | X:= [0-9] | X refers to major releases and Y to minor releases |
| | Y:=[0-9] | |
| format | raw summary | Indicates whether each record refers to raw nucleotide sequence data (i.e., individual sequencer reads) or summary STR data (i.e., summary of multiple reads) |
| caseID | [A-Za-z0-9_. :-] | Unique case identity numbers for case management |
| sampleID | [A-Za-z0-9_. :-] | Unique sample identity numbers for case management |
| labID | [A-Za-z0-9_. :-] | Unique laboratory identity numbers for case management |
| techID | [A-Za-z0-9_. :-] | Unique technician identity numbers for case management |

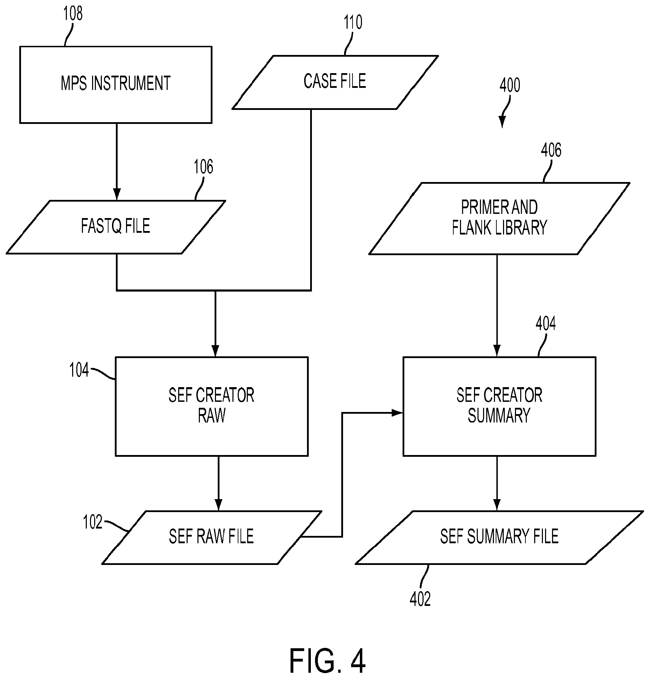

Referring now to FIG. 4, a method 400 of creating SEF files containing summary nucleotide sequence data and related metadata is shown is as a simplified flow diagram (along with elements 102-110 from the method 100, described above). The method 400 is intended for use nearer the end of the forensic DNA process and generates an SEF summary-format file 402. While SEF raw-format files 102 (like FASTQ files 106) may contain thousands to millions of records and be several gigaBytes in size, the SEF summary-format file 402 is immensely more compact (e.g., on the order of kiloBytes), yet includes the information necessary to substantiate the results of an MPS-based allelotyping workflow. As described further below, the SEF summary-format file 402 may contain a single record (i.e., four text lines) per locus for homozygous loci that do not exhibit stutter or artifacts. As another example, an SEF summary-format file 402 for an eighteen-locus profile with twelve homozygous loci, six heterozygous loci, and half of the alleles showing a stutter response may contain just forty-five records (i.e., 180 rows of human readable data).

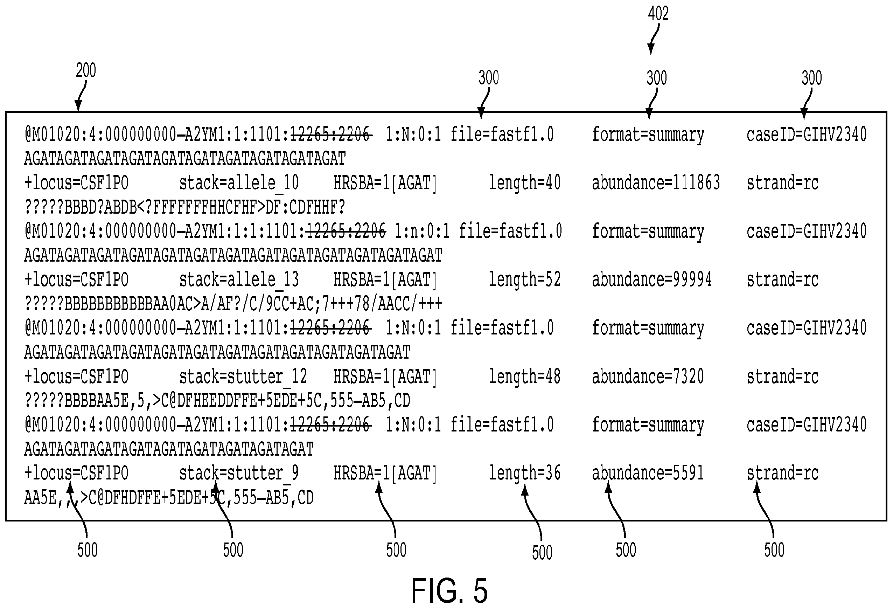

The SEF Creator Summary algorithm 404 ingests an SEF raw-format file 102 (e.g., generated by the SEF Creator Raw algorithm 104) and generates an SEF summary-format file 402. In order to perform this manipulation, the SEF Creator Summary algorithm 404 may call a library 406 containing nucleotide sequences that may be used for locus identification and grooming operations. The nucleotide sequences stored in the library 406 may include 5' primer sequences used to PCR amplify each STR locus, flanking sequences that are immediately 5' and 3' to each STR locus, and lists of canonical motifs found within each STR locus. The library 406 may be an updatable library 406, such that new information regarding the foregoing primer sequences, flanking sequences, and canonical motifs may be easily added and used by the algorithm 404. One illustrative example of an SEF summary-format file 402 that may be output by the algorithm 404 is shown in FIG. 5 (showing the first sixteen lines, i.e. four records, of the file 402). After the method 400, the SEF summary-format file 402 may be parsed into separate locus-specific files, may be graphed, may be used to generate CODIS-compliant allelotype records, and may be otherwise manipulated (e.g., using various SEFtools utility algorithms), if desired.

Generally, the SEF Creator Summary algorithm 404 generates the SEF summary-format file 402 from the SEF raw-format file 102 (or from a FASTQ file 106 or other raw file 102) by identifying all records of the raw file 102 that contain identical nucleotide sequence data representing a unique STR and grouping these records together to create the records of the summary file 402. After receiving the SEF raw-format file 102 (or other raw file 102), the algorithm 404 examines each record in the raw file 102 to determine whether a portion of the nucleotide sequence data (e.g., in the second text line of each record) represents an STR associated with a locus. In some examples, this determination may involve identifying whether a portion of the nucleotide sequence data of each record represents a 5' primer sequence used to amplify the locus. As noted above, the algorithm 404 may reference the library 406, which includes the primer sequences for each locus, when making this determination.

For each record in which a portion of the nucleotide sequence data is determined to represent an STR associated with a locus, the SEF Creator Summary algorithm 404 will then place that portion of the nucleotide sequence data representing the STR associated into a locus-specific list. This may involve trimming away portions of the nucleotide sequence data that do not represent the STR. In particular, in some examples, the algorithm 404 may remove portions of the nucleotide sequence data that represent a flanking sequence. Once again, the algorithm 404 may reference the library 406, which contains lists of flanking sequences, when trimming the nucleotide sequence data.

After the portions of nucleotide sequence data representing STRs are each placed into locus-specific lists, the SEF Creator Summary algorithm 404 will determine a number of occurrences within each locus-specific list of identical nucleotide sequence data representing a unique STR. In some examples, the algorithm 404 may compare the characters of each portion of nucleotide sequence data to all other portions in the same locus-specific list to find identical matches. This exact-matching scheme will result in a read count (i.e., a number of occurrences) for each even slightly different STR. For instance, where nucleotide sequence data reflects a single nucleotide polymorphism (SNP), the algorithm 404 will count this nucleotide sequence data as its own unique STR (and will not group it with a similar STR not exhibiting the SNP). It will be appreciated that this approach is quite different from prior alignment-based systems, which attempts to identify which reference sequence each portion of nucleotide sequence data most resembles. Such prior alignment-based system would typically lump the nucleotide sequence data reflecting the SNP in with similar sequences.

After the number of occurrences of each unique STR within each locus-specific list is determined, the SEF Creator Summary algorithm 404 will select which nucleotide sequence data to write to the summary file 402. This determination is made based on some abundance threshold, which may take a number of forms (and may be user-configurable, in some examples). For instance, the algorithm 404 may sort the unique STRs by relative number of occurrences and select a certain number of those at the top of the list for inclusion in the summary file 402. The remaining data, which essentially represents noise, will be discarded from the summary file 402 (but might still be retrieved from the raw file 102, if needed). In other examples, the abundance threshold may be embodied as a certain percentage threshold. For instance, all unique STRs that make up over 10% of the total reads for their locus-specific list might be included in the summary file 402. For each unique STR that is to be included in the summary file 402, the algorithm 404 will create one record in the summary file 402.

The SEF summary-format file 402 retains the human readability feature of all SEF files. This human readability allows direct use by adjudicating parties, who need evidentiary data presented in a way that is easily read and understood by lay audiences without the need for special software and bioinformatics expertise. As illustrated in FIG. 5, forensic metadata, in the form of attribute-value pairs 300, is presented in the first text line of each record of the file 402, appended to the unique sequence identifier 200 created by the MPS instrument 108. This information may be retained from the SEF raw-format file 102 that is input to the SEF Creator Summary algorithm 404. For illustration purposes, the x,y coordinates of the Illumina sequencing cluster are crossed out to indicate that these values would be replaced by null values because they are not relevant in the summary format. The second text line of each record of the SEF summary-format file 402 comprises a number of characters representing nucleotide sequence data corresponding to one of the unique STRs selected by the algorithm 404 (i.e., an STR for which a read count in the sample exceeded an abundance threshold). The third text line of each record of the file 402 may comprise STR metadata summarizing the read records associated with the corresponding unique STR. In the illustrative example of FIG. 5, the third text line of each record contains several attribute-value pairs 500, with each pair tab-separated. Several illustrative attribute-value pairs 500 are set forth in Table 3 below, including possible permissible values and intended purposes. The fourth text line of each record of the file 402 may comprise a number of characters representing average quality scores associated with the nucleotide sequence data of the second text line. These average quality scores may be a function of the quality scores of all reads summarized by that record and may be formatted as Phred quality scores. In other examples, the characters of the fourth text line may represent median quality scores associated with the nucleotide sequence data of the second text line.

| Table 3 | | |
|---|---|---|
| Attribute | Permissible Values | Purpose |
| locus | X:= [0-9] | X refers to the number of full motif repeats, Y refers to the |
| | Y:=[0-9] | number of nucleotides in a partial motif repeat |
| stack | allele _x[.y], | Identifies whether the read stack has been designated as an |
| | stutter_x[.y], artifact_x[.y] unassigned | allele, stutter, or an artifact (x[.y] denotes the ISFG allele designation) |
| hrsba | [[]0-9 ACTGNURYSW KMBDHVN.-] | Human readable sequence based allele designation (IUPAC nucleotide designations are used) |
| length | [0-9] | Length of nucleotide sequence data |
| abundance | [0-9] | Read counts corresponding to each read stack |
| strand | isfg rc | Refers to the strand represented by the nucleotide sequence data |
| at | [0-9] | Refers to the analytic threshold |

The portion of the SEF summary-format file 402 illustrated in FIG. 5 exemplifies data from a single locus (namely, CSF1PO) for a case where the subject sample is single-source and is heterozygous. As reflected in FIG. 5, the subject is heterozygous 10,13 at this locus (denoted by the "stack=allele_10" and "stack=allele_13" value-attribute pairs 500) and the data exhibit stutter corresponding to allele lengths 9 and 12 (denoted by the "stack=stutter_9" and "stack=stutter_10" attribute-value pairs 500). In this case, only the DNA strand designated as the reverse complement of the ISO-standard strand is included (denoted by the "strand=rc" attribute-value pair 500). The read counts corresponding to each read stack is indicated by the "abundance" attribute-value pair 500. It will be appreciated that several ratios used in forensic analysis may be calculated from these values, including the stack height ratio (aka allele-coverage ratio) and the stutter ratio. A human-readable sequence-based allele (HRSBA) designation is also present. The HRSBA designation is reflected in the "hrsba" and "length" attribute-value pairs 500.

The HRSBA designation contained in the third text line of each record of the SEF summary-format file 402 assists with the difficult task of reading repetitive DNA sequences and discerning the sometimes-subtle differences between them. HRSBA also provides a deterministic and non-arbitrary naming convention for sequence-defined alleles. Under current practice, STR alleles with identical nucleotide lengths but variant sequences are discriminated by denoting the allele length in International Society for Forensic Genetics (ISFG) X.Y format, followed by arbitrary notations such as letters, primes, or asterisks. These arbitrary notations fall short in two ways. First, they require the user to refer back to a lookup table for the actual sequence variations indicated by an asterisk or prime symbol. Second, without a complex and globally coordinated configuration management system, it is impossible to avoid inadvertent reuse of the same shorthand designations for different sequence variants by different laboratories. By contrast, the HRSBA designation disclosed herein has the advantage of determinism and clarity. By following a rule-based approach to generating HRSBA designations, every sequence variant will map to a unique HRSBA designation, allowing the underlying sequence to be easily derived from the HRSBA designation. The HRSBA designations may also be parsed by computer algorithms (e.g., SEFtools utility algorithms) and rendered into alternative formats, including the original nucleotide sequence data.

In the illustrative example, the HRSBA designation is reflected in two attribute-value pairs 500. First, the "hrsba" attribute-value pair 500 summarizes the STR sequence in the 5' to 3' direction, starting with the first nucleotide position in the STR sequence as nucleotide position 1. Nucleotide position numbers may be followed by the repeat motif bracketed (e.g., in square brackets) or by a single nucleotide code. It is contemplated that the HRSBA may use any type of punctuation to "bracket" a repeat motif. The nucleotide position refers to the position of the first nucleotide to the 3' of the integer. In the case of a repeat motif, it refers to the first nucleotide of the motif. For example, a non-variant CSF1PO locus normally begins with the designation 1[AGAT], where the 5' nucleotide A occupies nucleotide position number 1. The remaining three nucleotides (i.e., GAT) of the motif occupy sequence positions 2, 3 and 4, respectively. In the case of nucleotides not contained in a repeat motif, the positions of each nucleotide are denoted. For example, the TH01 allele 8.3 sequence listed by the National Institute of Standards and Technology (NIST) is as follows:
AATGAATGAATGAATGAATGATGAATGAATGAATG (SEQ ID NO: 1)
In the illustrative example, the HRSBA designation reflecting this sequence would be:
1[AATG]21ATG24[AATG] length=35 (SEQ ID NO: 1)
This HRSBA designation indicates that the 5' nucleotide A in the AATG repeat is the first nucleotide in the sequence, while the nucleotide A in the non-repeat motif ATG is in sequence position 21. Furthermore, the 5' nucleotide A in the second repeat motif is in sequence position 24, and the total length of the sequence is 35, as indicated by the "length" attribute-value pair 500. Thus, the 3' nucleotide G in the sequence is in sequence position 35. It will be appreciated from the foregoing that the HRSBA designation is unambiguous and deterministic for any sequence variant that might be encountered in actual sequencing. Any mutation can be accommodated, including complex ones such as segment inversions. Unlike the alignment-based methods typically used in current allelotyping processes, the HRSBA designation in not dependent upon reference sequences. Rather, it is reference independent and will not be affected by possible future changes to reference sequences.

As noted above, the value of the "length" attribute-value pair 500 denotes the total length of nucleotides comprising the STR sequence. This attribute-value pair 500 serves two purposes. First, it denotes the end of the STR sequence and therefore defines the number of repeats of the ending canonical motif of the sequence. Second, the length attribute-value pair 500 quickly identifies the allele length in nucleotides by visual inspection. Combined with the "locus" attribute-value pair, this information may be used to derive the ISFG allele designation, although this information is also encoded by the value of the "stack" attribute-value pair 500 in cases where the stack corresponds to an allele.

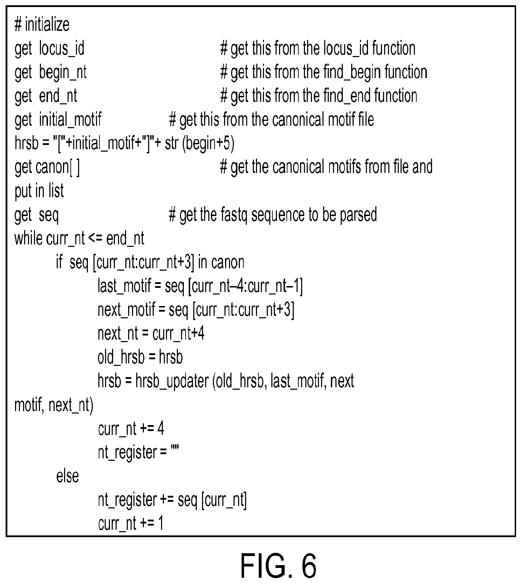

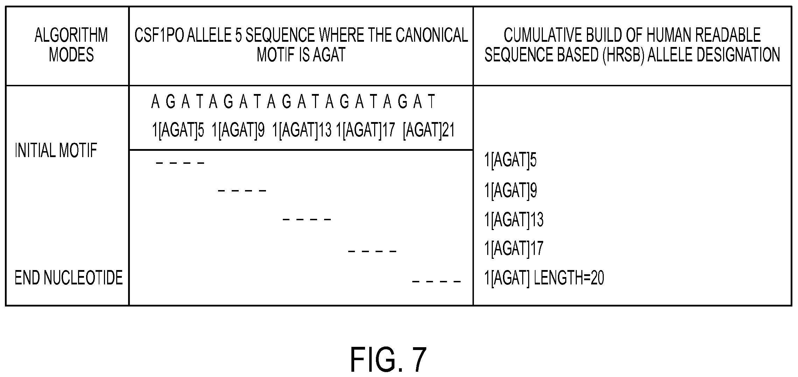

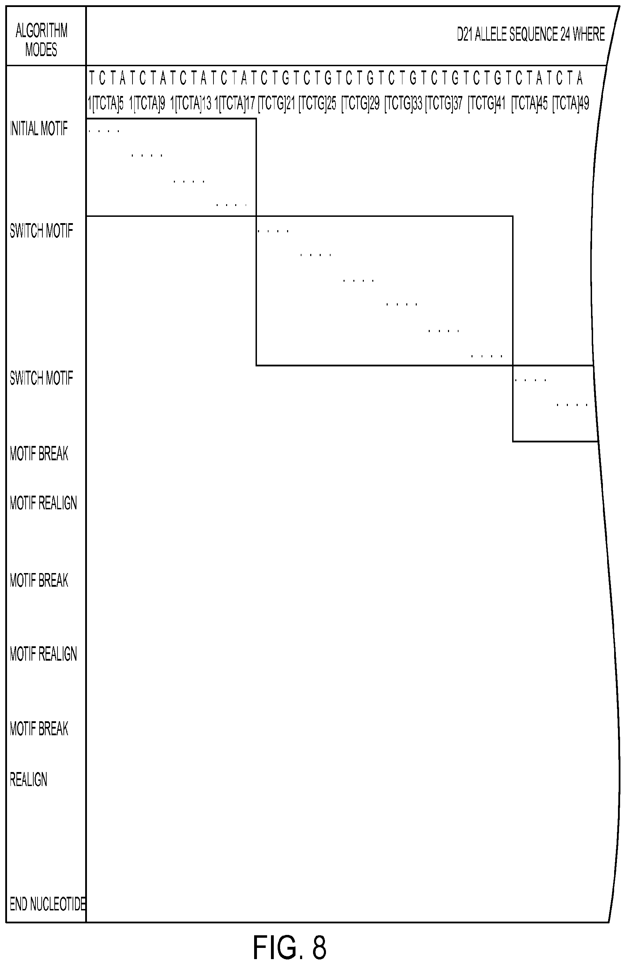

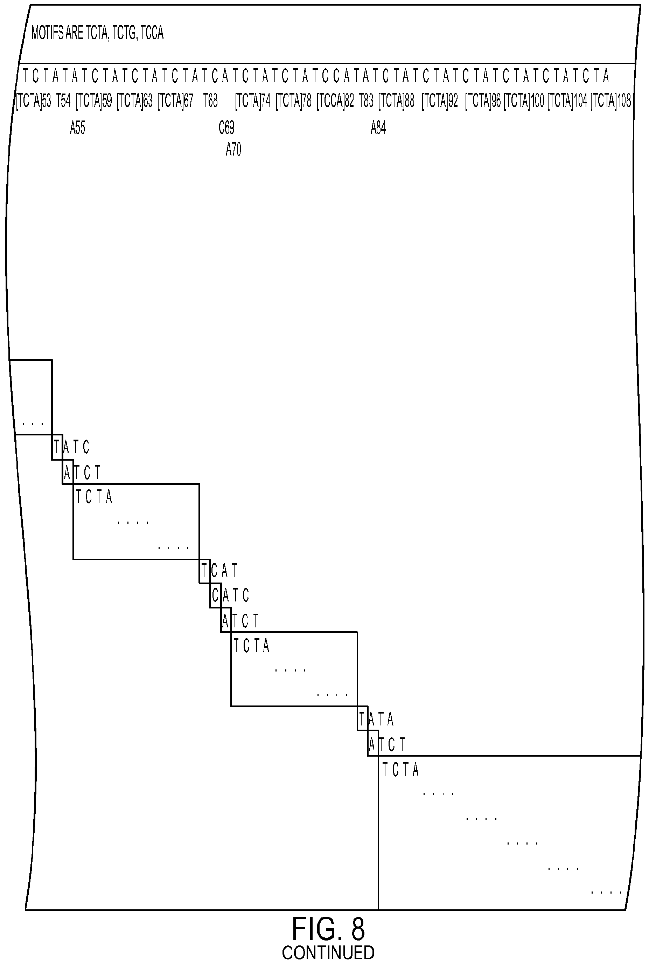

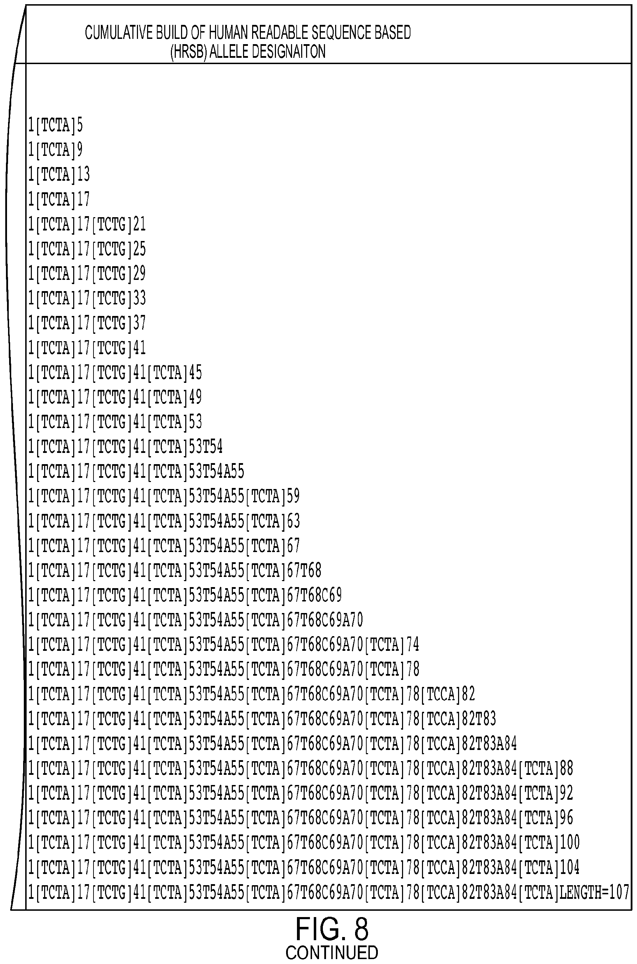

In the illustrative example, the HRSBA designation described above is generated using a motif aware allellotyper (MAA) algorithm, which may form part of the SEF Creator Summary algorithm 404. Pseudo-code for the MAA algorithm is presented in FIG. 6, while two examples of the operation of the MAA algorithm (described in detail below) are illustrated graphically in FIGS. 7 and 8. The MAA algorithm uses the portions of nucleotide sequence data determined to correspond to unique STRs, as described above, to generate the HRSBA designations corresponding to each unique STR. These HRSBA designations may then be included in each record of the SEF summary-format file 402. The MAA algorithm leverages prior knowledge about the genetics and genomics of STR loci, particularly the facts that (1) the STR loci selected for use in forensic analysis exhibit a limited repertoire of repeat motifs within their alleles (i.e., the "canonical motifs" for that locus), (2) all alleles of a given forensic STR loci start with a specific initial repeat motif (i.e., a "canonical opening motif" for that locus), and (3) all alleles of a given forensic STR loci end with a specific final repeat motif (i.e., a "canonical ending motif" for that locus).

The illustrative example of the MAA algorithm uses the ISFG convention that the initial repeat sequence motif be set equal to the first 5' nucleotides that can define a repeat motif. Using this information, the deterministic HRSBA designation can be generated for each allele regardless of sequence. The MAA algorithm initializes on the occurrence of a canonical opening motif (see Table 4 below) immediately following the unique 5' flanking sequence. In this example, mutations in either of the 5' flanking region or the initial repeat motif will result in allele dropout but not in an aberrant allele call, due to the deterministic property of the algorithm. In some examples, users may have the option of manually examining reads corresponding to loci exhibiting allele dropout.

| Table 4 | | | |
|---|---|---|---|
| Locus | Canonical Opening Motif | Canonical Ending Motif | Set of Canonical Motifs |
| CSF1PO | AGAT | AGAT | AGAT |
| FGA | TTTC | TTCC | TTTC, TTTTTT, CTTT, CTCC, TTCC, CTTC, CCTT |
| TH01 | AATG | AATG | AATG |
| TPOX | AATG | AATG | AATG |
| vWA | TCTA | TCTA | TCTA, TCTG, TCCA |
| D3S1358 | TCTA | TCTA | TCTA, TCTG |
| D5S818 | AGAT | AGAT | AGAT |
| D7S820 | GATA | GATA | GATA |
| D8S1179 | TCTA | TCTA | TCTA, TCTG |
| D13S317 | TATC | TATC | TATC |
| D16S539 | GATA | GATA | GATA |
| D18S51 | AGAA | AGAA | AGAA |
| D21S11 | TCTA | TCTA | TCTA, TCTG, TCCA |

Once initialized, the MAA algorithm utilizes a motif-aware 5' to 3' sliding window to parse the nucleotide sequence data of each allele. The sliding window follows two movement rules. First, if a canonical motif is present in the sliding window, a bracketing function is implemented for the canonical motif and then the sliding window moves four nucleotides to the right (3'). For the first instance of a canonical motif, the bracketing function adds the nucleotide bases in the sliding window to the HRSBA. In subsequent instances of the same canonical motif that is immediately adjacent to a prior instance, the bracketing function merely increments the nucleotide position to the immediate 3' side of the canonical motif. Second, if a canonical motif is not found in the sliding window, the first nucleotide base in the window is pushed into a register and then the sliding window moves one position. This one-position movement of the four-nucleotide sliding window continues until the four-nucleotide sliding window encounters a canonical motif for that locus. When that occurs, the MAA algorithm again proceeds in four-position increments.

This movement of the sliding window continues until the final nucleotide of the STR allele sequence is encountered. The MAA algorithm performs a quality check to be sure the sequence ends in a canonical ending motif, generating a user alert otherwise. While the foregoing description uses the illustrative example of a four-nucleotide sliding window (to target tetramers), it is contemplated that the sliding window may be of any size. By way of example, the MAA algorithm may utilize a five-nucleotide sliding window to target pentameric repeats, such as those found in the pentaD and pentaE loci. In some examples, the MAA algorithm may switch to using a five-nucleotide sliding window when one of these loci is detected based on the uniquely-identifying 5' primer sequence.

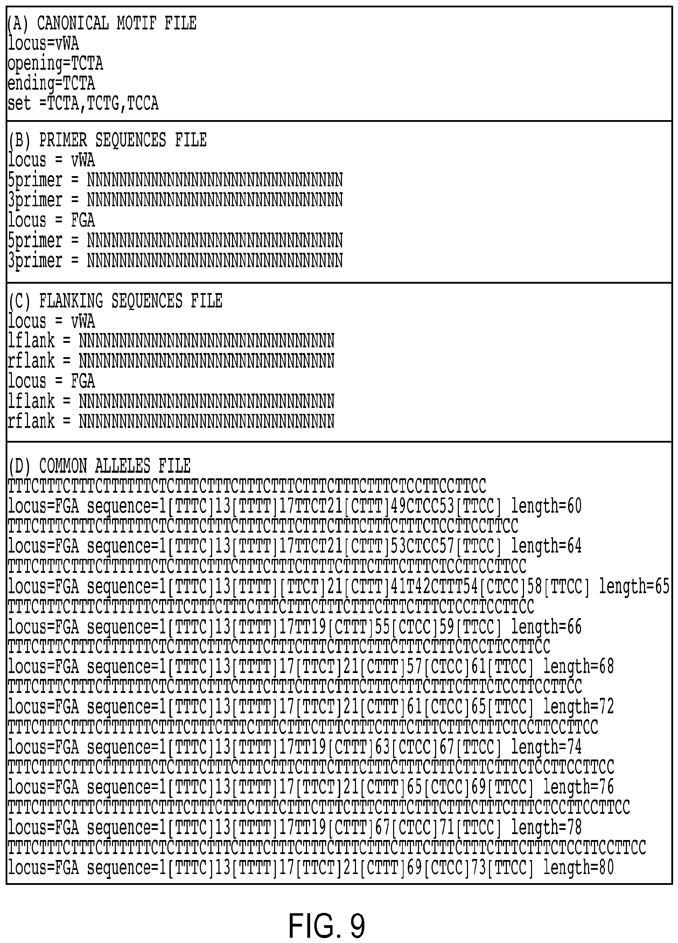

During operation, the MAA algorithm may call the library 406 described above to reference the canonical motifs for a particular locus. FIG. 9 shows a portion of one illustrative look-up table that may be contained in the library 406. As shown in FIG. 9 (part a), the library 406 may include a list of the canonical motifs associated with particular loci. The library 406 may also contain a list of primer sequences and flanking sequences for particular loci, as shown in FIG. 9 (parts b and c, respectively). In some examples, the library 406 may further contain lists of the HRSBA designations for common alleles (FIG. 9, part d). This feature may improve the speed of software implementing the MAA algorithm. By way of example, if a given allele sequence is identical to one of the "common alleles," then a pre-computed HRSBA designation can simply be retrieved from a lookup table as opposed to constructing it de-novo with the MAA algorithm.

Reference to the external library 406 allows routine updating of information about forensic STR loci, without changes to the underlying software code implementing the MAA algorithm. The discovery of previously unknown allele sequences may be anticipated as the number of individuals sequenced increases. Some of these sequences may be observed frequently enough to be included in the library 406 as common alleles. Similarly, the canonical motifs used by the MAA algorithm may be updated in the future if additional repeat motifs are discovered for a given locus. Thus, the updatable library 406 allows software implementing the MAA algorithm to remain relevant and accurate.

As mentioned above, the present disclosure also relates to methods of allelotyping loci using an approach based on matching and sorting of the reads generated by an MPS instrument when analyzing a sample. STR loci exist in multiple states, called alleles. STR alleles always differ from one another by nucleotide sequence. In addition, alleles often differ from one another by sequence length. Allelotyping is the process of identifying the one or more alleles present at a particular STR locus in a given sample (by way of example, for a sample from one human, the one allele where the locus is homozygous or the two alleles where the locus is heterozygous). The FBI and other law enforcement agencies around the world have selected about twenty-four specific STR loci for use in forensic DNA analysis applications. These STR loci have been selected because they are highly polymorphic, i.e., multiple alleles exist within the relevant populations. For example, the thirteen STR loci considered by the FBI as their "core" loci each exhibit eight to ten different common allelic forms.

As described above, a typical MPS process starts with a DNA sample, which is typically amplified using PCR (however, it will be appreciated that certain DNA samples can also be directly sequenced by MPS instruments without pre-amplification). The output of the MPS process typically takes the form of one or more computer files containing text strings representing the nucleotide sequence that were read by the MPS instrument. The parallel nature of MPS results in numerous replicates of the nucleotide sequence of each allele. This is particularly true when the DNA is pre-amplified by PCR, where the number of replicates of each allelic sequence may number in the tens of thousands. However, both the PCR pre-amplification process and the sequencing process itself have non-trivial error rates. Errors are realized as incorrect nucleotide sequences in many of the copies of the original DNA sequences, i.e., the "true" alleles that existed in the original DNA sample. These sequence errors may act to obscure the nucleotide sequences of the "true" alleles.

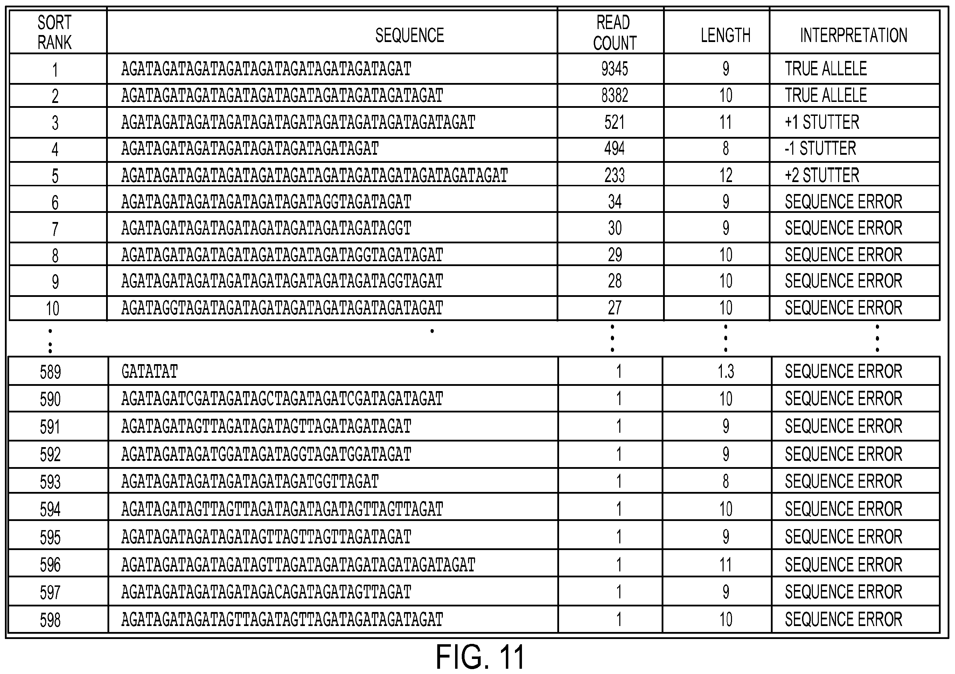

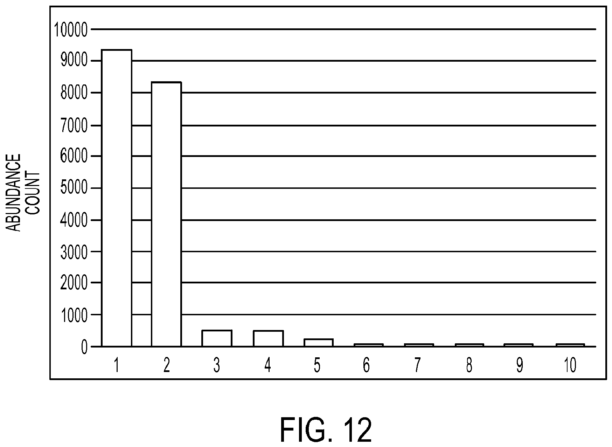

The diversity of unique nucleotide sequences generated by a PCR-MPS workflow can be illustrated using an experiment performed on a DNA sample from an anonymous human subject. This anonymous human subject was known to contain allele numbers 9 and 10 (with known sequences [AGAT]₉ (SEQ ID NO: 2) and [AGAT]₁₀ (SEQ ID NO: 3), respectively) at the CSF1PO locus. The MPS instrument returned approximately 20,000 replicate sequences for the two alleles at this locus (i.e., an average of 10,000 replicate sequences per allele). In the absence of error, only these two "true" allele nucleotide sequences would be returned in the data generated by the MPS instrument. However, because of error in the PCR amplification and MPS processes, a total of 598 unique nucleotide sequences were observed. Two correspond to the "true" alleles, and the remaining 596 correspond to method artifacts and sequencing errors. FIG. 11 shows twenty of the 598 unique nucleotide sequences output by the MPS instrument (the top ten most abundant nucleotide sequences, and ten "singleton" nucleotide sequences that each occurred only once), while FIG. 12 shows a graph of the number of reads by the MPS instrument for the top ten most abundant nucleotide sequences. The two most abundant unique nucleotide sequences correspond to the "true" alleles (i.e., allele numbers 9 and 10). The next most abundant unique sequences correspond to PCR stutter artifacts (i.e., the insertion or deletion of repeat motifs in nucleotide sequences due to strand slippage in the PCR process). The less abundant sequences exhibit a wide range of mutations, including sequence truncations and base substitution errors.

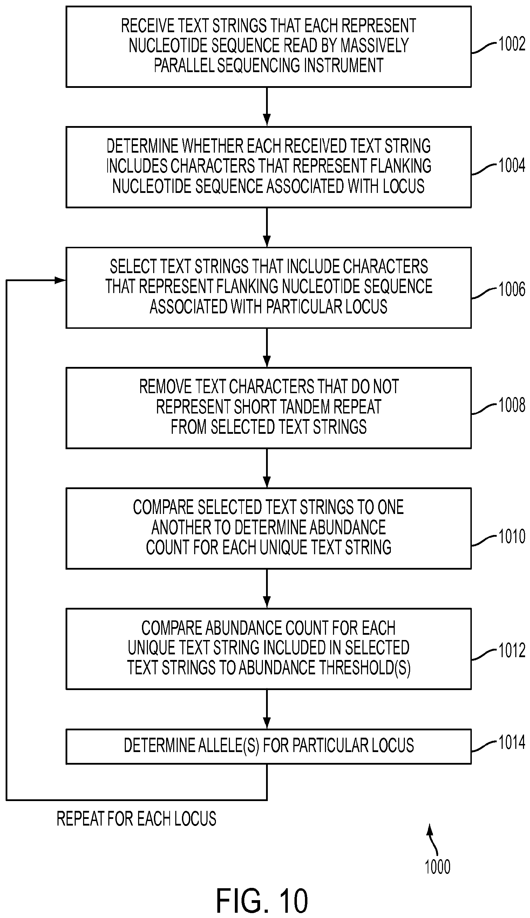

Referring now to FIG. 10, one illustrative example of an allelotyping method 1000 that may be used to determine the one or more "true" alleles for each locus of a sample analyzed by an MPS instrument is shown as a simplified flow diagram. In some examples, the allelotyping method 1000 may be implemented as one or more software routines executed by one or more processors. By way of example, it is contemplated that the allelotyping method 1000 may be performed by a processor of the MPS instrument analyzing the sample, in some examples, or by a processor of a separate computing device that receives nucleotide sequence data output by the MPS instrument (for instance, in the form of a FASTA file, a FASTQ file, or an SEF raw-format file 102), in other examples. The allelotyping method 1000 is illustrated in FIG. 10 as a number of blocks 1002-1014, which will be described in detail below. It will be appreciated that, although the allelotyping method 1000 is generally shown in FIG. 10 and described below as having a linear workflow, the allelotyping method 1000 may be implemented using any number of workflows (including, by way of example, workflows in which various portions of the allelotyping method 1000 are performed in parallel).

The allelotyping method 1000 begins with block 1002 in which a number of text strings that each represent a nucleotide sequence that was read by an MPS instrument are received. In these text strings, each character represents one base of a nucleotide sequence read by the MPS instrument. As noted above, due to the parallel nature of the MPS process, one sample may result in tens or hundreds of thousands of reads and, hence, associated text strings. Many MPS instruments output this data in files that follow either the FASTA or FASTQ format. As such, in some examples of the allelotyping method 1000, block 1002 may involve receiving such a FASTA or FASTQ file and reading a number of text strings that each represent a nucleotide sequence from that file. In other examples, block 1002 may involve receiving files in different formats (other than FASTA or FASTQ).

After block 1002, the allelotyping method 1000 proceeds to block 1004 in which a bioinformatic matching procedure is used to determine which reads from the MPS instrument (represented by the received text strings) correspond to one or more particular loci present in the sample. In the illustrative example of block 1004, each of the received text strings is evaluated to determine whether it includes characters that represent a known flanking nucleotide sequence associated with a particular locus. The nucleotide sequence of each STR locus is generally flanked, on both the 5' and 3' ends, by particular flanking nucleotide sequences. These complex nucleotide sequences can be rare or unique in the DNA analyzed by the MPS instrument. As such, the presence of one (or both) of these flanking nucleotide sequences may indicate the presence of an STR locus in the nucleotide sequence represented by the text string. It is contemplated that, in other examples, the text strings may be searched for other specific nucleotide sequences (e.g., the 5' primer sequences used to PCR amplify each STR locus).

The flanking nucleotide sequences, or other specific nucleotide sequences, used in block 1004 may be any length. Longer sequences are more likely to be unique, but increasing length should be balanced against the slower bioinformatic processing inherent to long-sequence matching routines. Moreover, the longer a sequence, the more likely that reads which should contain the sequence will contain an error, reducing the sequence's value as an identifier. In some examples, block 1004 may reference an external, updatable library of flanking nucleotide sequences (or other specific nucleotide sequences). The text string matching routine used in block 1004 can be stringent (e.g., exact character matching) or flexible (e.g., close, overall matching). Block 1004 may use any number of bioinformatic approaches, including, but not limited to, string matching routines built into common programming languages (e.g., Java, C++, Perl or Python), regular expression routines built into some programming languages (e.g., Perl) or available as modules for other programming languages, or the like. In some examples of block 1004, each text string determined to correspond to a particular locus may be grouped or segregated by locus. In other examples, each text string determined to correspond to a particular locus may be metadata tagged with that particular locus. In some examples, text strings that are not identified as corresponding to any locus (e.g., where the text string does not contain a known flanking nucleotide sequence) may be discarded.

After block 1004, the allelotyping method 1000 proceeds to block 1006 in which the text strings representing nucleotide sequences associated with a particular locus are selected for further processing. In other words, blocks 1006-1014 of the allelotyping method 1000 are performed on a locus-by-locus basis. As indicated in the illustrative example of FIG. 10, blocks 1006-1014 may be repeated for each locus identified as present in the sample in block 1004. In some examples, block 1006 may involve selecting a locus-specific list containing all of the text strings determined in block 1004 to contain characters that represent a known flanking nucleotide sequence associated with a particular locus. In other examples, block 1006 may involve selecting all text strings assigned a particular metadata tag in block 1004.

After block 1006, the allelotyping method 1000 proceeds to block 1008 in which each of the text strings selected in block 1006 are trimmed to remove characters that do not represent the nucleotide sequence of interest. For instance, in some examples, all characters except those representing an STR may be removed from each of the selected test strings. In other examples, all characters except those representing a nucleotide sequence containing a SNP may be removed from each of the selected test strings. In the illustrative example of block 1008, the characters representing the 5' and 3' flanking nucleotide sequences (used to identify the text string in block 1004), as well as any characters outside of the characters representing the 5' and 3' flanking nucleotide sequences, are removed from each of the selected test strings. After block 1008, the text strings contain only the alleles proper, but remain identified with a particular locus (due to the grouping, metadata tagging, or other segregation procedure performed in block 1004). It is contemplated that, in some examples of the allelotyping method 1000, block 1008 may be omitted, keeping the characters that do not represent the nucleotide sequence of interest but ignoring those characters in blocks 1010-1014.

After block 1008, the allelotyping method 1000 proceeds to block 1010 in which each unique text string included in the selected text strings (associated with a particular locus) are grouped and counted. In other words, block 1010 determines an abundance count for each nucleotide sequence represented by the selected text strings. In the illustrative example, block 1010 involves comparing the selected text strings to one another to determine each unique text string and its abundance in the group of selected text strings. Block 1010 may be performed by applying a stringent text string matching routine (e.g., exact character matching) to the selected text strings. As noted above, it is contemplated that string matching routines built into common programming languages (e.g., Java, C++, Perl or Python), regular expression routines built into some programming languages (e.g., Perl) or available as modules for other programming languages, or the like. Block 1010 may also involve sorting the unique text strings, each representing a unique nucleotide sequence read by the MPS instrument, into a list according to their abundance counts. FIG. 11 illustrates top and bottom portions of such a list, in which the 598 unique nucleotide sequences output by an MPS instrument and determined to be associated with the CSF1PO locus were sorted by abundance count.

It will be appreciated that the exact-matching scheme in block 1010 will result in an abundance count (i.e., a number of occurrences) for each even slightly different nucleotide sequence. For instance, where a text string represents a nucleotide sequence containing a SNP, the allelotyping method 1000 will independently count this unique text string (and will not group it with a different text string representing a similar nucleotide sequence not exhibiting the SNP). This approach is quite different from prior alignment-based allelotyping methods, which attempt to identify which reference sequence each portion of nucleotide sequence data most resembles.

After block 1010, the allelotyping method 1000 proceeds to block 1012 in which the abundance count for each unique text string determined in block 1010 is compared to one or more abundance thresholds. As noted, while the MPS workflow results in many unique nucleotide sequences for a particular locus, one or two of these nucleotide sequences will be the "true" alleles for that particular locus (in the case of a sample from one human). As explained further below, comparison of each of the abundance counts determined in block 1010 to an abundance threshold may allow determination of the one or more alleles for that particular locus. In the illustrative example, block 1012 involves identifying the unique text string having the highest abundance count from among the selected text strings. The nucleotide sequence represented by this text string will be one allele for the associated locus, because despite the non-trivial error rates, PCR and MPS workflows exhibit sufficient fidelity to generate correct sequences in abundance. The illustrative example of block 1012 also involves calculating a ratio of the abundance count for each unique text string as compared to that highest abundance count. It may then be determined whether the ratio calculated for each unique text string exceeds the abundance threshold(s). In some examples of block 1012, each abundance threshold may be a predetermined percentage value. For instance, the abundance threshold may be a percentage value in the range of 15% to 60%, as explained further below. In other examples, the abundance threshold may be a percentage value that is configurable by a user of the allelotyping method 1000.

After block 1012, the allelotyping method 1000 proceeds to block 1014 in which the one or more alleles for the particular locus being considered are determined based on the comparisons performed in block 1012. By way of example, where the sample analyzed by the MPS instrument is known to contain DNA from a single human source, the abundance threshold used in block 1012 may be set to a percentage value in the range of about 50% to about 60%. It is known that, for sister alleles at a heterozygous locus, the lesser abundant allele will typically have a ratio of about 50-60% or greater to the more abundant allele (i.e., the allele represented by the text string with the highest abundance count). As such, if a second text string is determined in block 1012 to have an abundance count exceeding about 50-60% of the highest abundance count, block 1014 may conclude that the locus is heterozygous and that the second text string represents the lesser abundant sister allele. Alternatively, if no text string is determined in block 1012 to have an abundance count exceeding about 50-60% of the highest abundance count, block 1014 may conclude that the locus is homozygous and that the text string with the highest abundance count represents the only allele at that particular locus. As another example, in cases where it is unknown if the sample analyzed by the MPS instrument contained DNA from multiple sources, the abundance threshold used in block 1012 may be set to a percentage value of about 15% (or higher). This abundance threshold may capture alleles from a secondary source that is present in a lower amount in the sample, while avoiding detection of artifacts caused by PCR stutter (which tend to be about 4-15% of the corresponding allele abundance count). In yet another example, the abundance threshold might be set to a percentage value of about 4% to capture PCR stutter artifacts but avoid background noise (which tends to be < 4% of the highest abundance count).

In some illustrative examples, the allelotyping method 1000 of FIG. 10 may be used as part of the method 400 of FIG. 4 (e.g., as part of the SEF Creator Summary algorithm 404) to create SEF files 402 containing summary nucleotide sequence data and related metadata. For instance, the SEF Creator Summary algorithm 404 may perform the allelotyping method 1000 on raw nucleotide sequence data ingested from an SEF raw-format file 102 (or from a FASTQ file 106) to generate an SEF summary-format file 402. In such examples, block 1002 of the allelotyping method 1000 may involve receiving the SEF raw-format file 102 (or a FASTQ file 106), which contains a number of text strings that each represent a nucleotide sequence read by the MPS instrument 108. Upon completion of the allelotyping method 1000, the SEF Creator Summary algorithm 404 may write each unique text string determined in block 1012 of the allelotyping method 1000 to exceed the abundance threshold to the SEF summary-format file 402. In some examples, the algorithm 404 may write each unique text string determined in block 1014 of the allelotyping method 1000 to represent an allele for a particular locus to the SEF summary-format file 402.

It will be appreciated that, as a result of the allelotyping method 1000, the SEF summary-format file 402 generated by the SEF Creator Summary algorithm 404 may have a file size that is significantly smaller than that of the SEF raw-format file 102 (or the FASTQ file 106). While the raw files 102, 106 contain an individual, four-line record for every read performed by the MPS instrument 108, the SEF summary-format file 402 contains only one four-line record for each "true" allele in the sample (and any other nucleotide sequences of interest). As such, using the allelotyping method 1000, the algorithm 404 is able to "compress" the SEF raw-format file 102 (or the FASTQ file 106) into the SEF summary-format file 402 while retaining all of the information important for forensic analysis. Table 5 below sets forth five examples of FASTQ files 106 that were processed by the algorithm 404 to generate SEF files 402. As can be seen from these five examples, the allelotyping method 1000 can produce an SEF file 402 with a file size that is at least 10,000 times smaller than the file size of the source FASTQ file 106.

| Table 5 | | | | |
|---|---|---|---|---|
| FASTQ File Size (kB) | SEF File Size (kB) | Flank Size (nucleotides) | Min. Stack Coverage | Processing Time (Min.) |
| 1,723,996 | 20 | 10,10 | 4 | 2:05 |
| 697,339 | 18 | 10,10 | 4 | 0:45 |
| 697,339 | 12 | 10,10 | 50 | 0:39 |
| 1,794,807 | 13 | 10,10 | 50 | 2:04 |
| 1,414,563 | 11 | 10,10 | 50 | 1:37 |

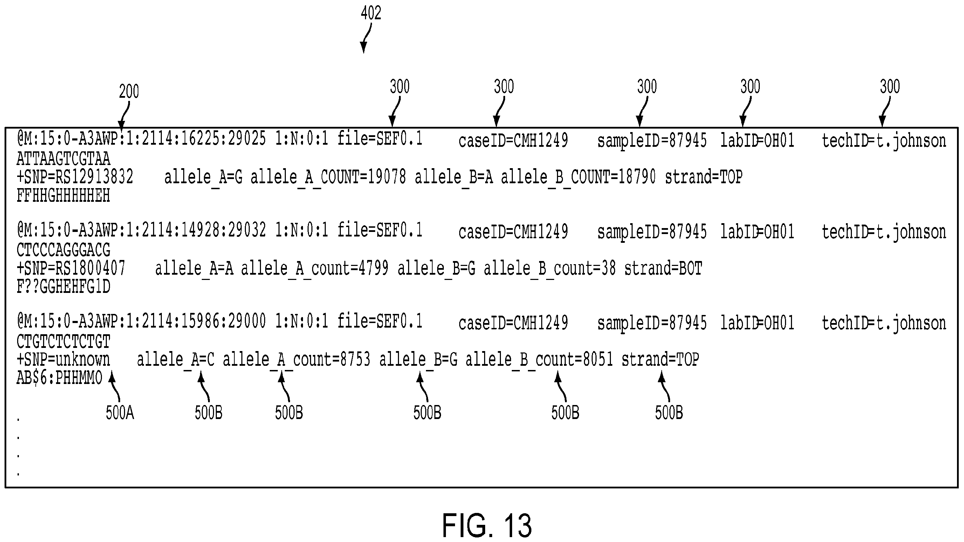

Several records of another illustrative example of an SEF file 402 are illustrated in FIG. 13. Specifically, the illustrative example of the SEF file 402 shown in FIG. 13 comprises data and metadata relating to several nucleotide sequences that contain a SNP. The SEF file 402 of FIG. 13 conforms to the file format specification outlined in Table 6, which references the FASTQ file format specification (the third column denoting several distinguishing features of this SEF file 402, as compared to FASTQ files 106). It will be appreciated that, in other examples, SEF files 402 may be formatted according to additional or different specifications than those set forth in Table 6.

| Table 6 | | |
|---|---|---|
| Line Type | FASTQ | SEF (Differences from FASTQ) |
| 1) Title and Description | First character must be "@" | • The systematic identifier produced by MPS instrument/software may comprise the initial portion of this field |
| | • Free format field with no length limitation | |
| | • Arbitrary content can be included | • Forensic metadata as a series of optional attribute-value pairs |
| 2) Sequence | • No specific initial character is required for this line type | • Only International Union of Pure and Applied Chemistry (ILTPAC) nucleotide base codes are permitted ("ACTGNURYSWKMBDHVN.-") |
| | • Any printable character is permitted | |
| | • Upper case, lower case, and mixed case accepted | |
| | • Can be line wrapped | |
| 3) End of Sequence | • First character must be "+" | • Content after "+" does not have to match the title line |
| | • If the title line is repeated, it must be identical | |
| | | • Human-readable allele designation for SNP or STR alleles as a series of optional attribute-value pairs |
| 4) Quality Scores | • Accepts printable ASCII characters 33-126 | • Averaged PHRED scores with an ASCII offset of 33 |
| | • Can be line wrapped | |
| | • Length must be equal to sequence length | |

Similar to FASTQ files, the SEF file 402 of FIG. 13 comprises a number of records, where each record includes data in four text lines. The second text line of each record contains a text string representing a nucleotide sequence containing a SNP. In some examples, this text string in the second line of each record of the SEF file 402 may be an output of the allelotyping method 1000 used to identify the SNP alleles. In the illustrative example of FIG. 13, the SNP nucleotide is printed as a lower case character flanked by upper case characters for the remainder of the nucleotide sequence. The fourth text line of each record may contain average quality scores for the nucleotide sequences represented by the text string of the second text line. The first and third text lines of each record may contain metadata related to case management and to evidence preservation.

The forensic metadata included in the first text line of each record of the SEF file 402 may be in the form of one or more attribute-value pairs 300. In the illustrative example of FIG. 13, the first text line of each record contains several attribute-value pairs 300. In some examples, the attribute-value pairs 300 may specify one or more of a file format, a unique case identifier, a unique sample identifier, a unique laboratory identifier, and a unique technician identifier associated with the SEF file 402. Several illustrative attribute-value pairs 300, including possible permissible values and intended purposes, are set forth in Table 2 above. As noted above, the first text line of each record of the SEF file 402 may also include a unique sequence identifier 200 created by the MPS instrument 108.

In the illustrative example of FIG. 13, the third text line of each record of the SEF file 402 comprises a human-readable allele designation for the SNP in the form of a number of attribute-value pairs 500A, 500B. Several illustrative attribute-value pairs 500A, 500B that may be used as part of the human-readable allele designation for the SNP are set forth in Table 7 below, including possible permissible values and intended purposes. The human readable allele designation for the SNP of each record strikes a balance between human and computer readability. One attribute-value pair 500A of the human readable allele designation for the SNP may specify a reference SNP identifier, as reported in the National Center for Biotechnology Information (NCBI) SNP database. Where no reference SNP identifier is available, this attribute-value pair 500A may specify "unknown." Additionally, a series of attribute-value pairs 500B of the human readable allele designation for the SNP may specify the state of the allele. For instance, the human readable allele designation may include attribute-value pairs 500B that specify a first SNP state, a second SNP state, an abundance count of the first SNP state, an abundance count of the second SNP state, and a strand of the nucleotide sequence represented in the second text line. In the illustrative example, the series of attribute-value pairs 500B that specify the state of the allele follow the conventions described in Illumina, Inc., "Technical Note: 'TOP/BOT' Strand and 'A/B' Allele," Pub. No. 370-2006-018 (June 27, 2006).

| Table 7 | | |
|---|---|---|
| Attribute | Permissible Values | Purpose |
| SNP | [A-Za-z0-9] | Reference SNP identifier, as reported in NCBI SNP database (can be "unknown") |
| allele_A | [G,A,T,C,g,a,t,c] | SNP allele state (following Illumina A/B convention) |
| allele_B | [G,A,T,C,g,a,t,c] | SNP allele state (following Illumina A/B convention) |
| Strand | [A-Za-z] | Strand where SNP is found (following Illumina TOPBOT convention). |
| allele_A_count | [0-9] | Abundance count for allele_A |
| allele_B_count | [0-9] | Abundance count for allele_B |

## Claims

1. A computer-implemented allelotyping method (1000) comprising:
selecting (1006), from among a group of text strings that each represent a nucleotide sequence that was read by a massively parallel sequencing (MPS) instrument, a plurality of text strings for which the nucleotide sequences represented by the selected plurality of text strings each correspond to a particular locus;
comparing (1010) the selected plurality of text strings to one another to determine an abundance count for each unique text string included in the selected plurality of text strings; and
determining (1014) one or more alleles for the particular locus by comparing the abundance count for each unique text string included in the selected plurality of text strings to an abundance threshold.

2. The allelotyping (1000) method of claim 1, wherein determining (1014) the one or more alleles for the particular locus comprises identifying one or more unique text strings that each represent a nucleotide sequence containing a short tandem repeat (STR).

3. The allelotyping (1000) method of claim 1, wherein determining (1014) the one or more alleles for the particular locus comprises identifying one or more unique text strings that each represent a nucleotide sequence containing a single nucleotide polymorphism (SNP).

4. The allelotyping (1000) method of any one of claims 1-3, wherein comparing the abundance count for each unique text string included in the selected plurality of text strings to the abundance threshold comprises identifying the unique text string having a highest abundance count from among the selected plurality of text strings.

5. The allelotyping (1000) method of claim 4, wherein comparing the abundance count for each unique text string included in the selected plurality of text strings to the abundance threshold further comprises calculating whether a ratio of the abundance count for each unique text string compared to the highest abundance count exceeds the abundance threshold.

6. The allelotyping (1000) method of claim 5, wherein the abundance threshold is a percentage value in the range of 15% to 60%.

7. The allelotyping (1000) method of claim 5, wherein the abundance threshold is a percentage value configurable by a user.

8. The allelotyping (1000) method of any one of claims 5-7, further comprising:
receiving (1002) a first text-based computer file comprising a group of text strings that each represent a nucleotide sequence that was read by the MPS instrument, prior to selecting the plurality of text strings for which the represented nucleotide sequences each correspond to the particular locus; and
generating a second text-based computer file comprising each unique text string for which the ratio exceeds the abundance threshold, wherein a file size of the second text-based computer file is smaller than a file size of the first text-based computer file.

9. The allelotyping (1000) method of claim 8, wherein the second text-based computer file comprises one or more unique text strings that each represent a nucleotide sequence determined to be an allele for the particular locus.

10. The allelotyping (1000) method of claim 8, wherein the file size of the second text-based computer file is at least ten-thousand times smaller than the file size of the first text-based computer file.

11. The allelotyping (1000) method of any one of claims 1-10, wherein the steps of (i) selecting (1006) the plurality of text strings for which the represented nucleotide sequences each correspond to a particular locus, (ii) comparing (1010) the selected plurality of text strings to one another to determine the abundance count for each unique text string included in the selected plurality of text strings, and (iii) determining (1014) one or more alleles for the particular locus by comparing the abundance counts to the abundance threshold are performed for each of a plurality of loci present in a sample that was read by the MPS instrument.

12. The allelotyping (1000) method of claim 11, wherein selecting the plurality of text strings for which the represented nucleotide sequences each correspond to one of the plurality of loci comprises:
Determining (1004) whether each of a plurality of text strings generated by the MPS instrument when reading the sample includes text characters that represent a flanking nucleotide sequence associated with a particular locus; and
selecting a plurality of text strings that include the text characters that represent the flanking nucleotide sequence associated with the particular locus.

13. The allelotyping (1000) method of claim 12, further comprising removing the text characters that represent the flanking nucleotide sequence from each of the selected plurality of text strings prior to comparing the selected plurality of text strings to one another to determine the abundance count for each unique text string included in the selected plurality of text strings.

14. The allelotyping (1000) method of any one of claims 1-13, further comprising removing all text characters that do not represent a short tandem repeat (STR) from each of the selected plurality of text strings prior to comparing the selected plurality of text strings to one another to determine the abundance count for each unique text string included in the selected plurality of text strings.

## Patentansprüche

1. Computerimplementiertes Allelotypisierungsverfahren (1000), umfassend:
Auswählen (1006), aus einer Gruppe von Textzeichenfolgen, die jeweils eine Nukleotidsequenz darstellen, die durch ein Massively-Parallel-Sequencing(MPS-)Instrument gelesen wurde, einer Vielzahl von Textzeichenfolgen, für welche die Nukleotidsequenzen, die durch die ausgewählte Vielzahl von Textzeichenfolgen dargestellt sind, jeweils einem bestimmten Ort entsprechen;
Vergleichen (1010) der ausgewählten Vielzahl von Textzeichenfolgen miteinander, um eine Häufigkeitszahl für jede einmalige Textzeichenfolge zu bestimmen, die in der ausgewählten Vielzahl von Textzeichenfolgen beinhaltet ist; und
Bestimmen (1014) von einem oder mehreren Allelen für den bestimmten Ort durch Vergleichen der Häufigkeitszahl für jede einmalige Textzeichenfolge, die in der ausgewählten Vielzahl von Textzeichenfolgen beinhaltet ist, mit einem Häufigkeitsschwellenwert.

2. Allelotypisierungsverfahren (1000) nach Anspruch 1, wobei das Bestimmen (1014) des einen oder der mehreren Allele für den bestimmten Ort Identifizieren von einer oder mehreren einmaligen Textzeichenfolgen umfasst, die jeweils jede eine Nukleotidsequenz darstellen, die eine kurze Tandemwiederholung (STR) enthält.

3. Allelotypisierungsverfahren (1000) nach Anspruch 1, wobei das Bestimmen (1014) des einen oder der mehreren Allele für den bestimmten Ort Identifizieren von einer oder mehreren einmaligen Textzeichenfolgen umfasst, die jeweils eine Nukleotidsequenz darstellen, die einen einzelnen Nukleotidpolymorphismus (SNP) enthält.

4. Allelotypisierungsverfahren (1000) nach einem der Ansprüche 1-3, wobei das Vergleichen der Häufigkeitszahl für jede einmalige Textzeichenfolge, die in der ausgewählten Vielzahl von Textzeichenfolgen beinhaltet ist, mit dem Häufigkeitsschwellenwert Identifizieren der einmaligen Textzeichenfolge mit einer höchsten Häufigkeitszahl unter der ausgewählten Vielzahl von Textzeichenfolgen umfasst.

5. Allelotypisierungsverfahren (1000) nach Anspruch 4, wobei das Vergleichen der Häufigkeitszahl für jede einmalige Textzeichenfolge, die in der ausgewählten Vielzahl von Textzeichenfolgen beinhaltet ist, mit dem Häufigkeitsschwellenwert ferner Berechnen umfasst, ob ein Verhältnis der Häufigkeitszahl für jede einmalige Textzeichenfolge verglichen mit der höchsten Häufigkeitszahl den Häufigkeitsschwellenwert überschreitet.

6. Allelotypisierungsverfahren (1000) nach Anspruch 5, wobei der Häufigkeitsschwellenwert ein Prozentwert in dem Bereich von 15% bis 60% ist.

7. Allelotypisierungsverfahren (1000) nach Anspruch 5, wobei der Häufigkeitsschwellenwert ein Prozentwert ist, der durch einen Benutzer konfigurierbar ist.

8. Allelotypisierungsverfahren (1000) nach einem der Ansprüche 5-7, ferner umfassend:
Empfangen (1002) einer ersten textbasierten Computerdatei, die eine Gruppe von Textzeichenfolgen umfasst, die jeweils eine Nukleotidsequenz darstellen, die durch das MPS-Instrument gelesen wurde, vor dem Auswählen der Vielzahl von Textzeichenfolgen, für welche die dargestellten Nukleotidsequenzen jeweils dem bestimmten Ort entsprechen; und
Erzeugen einer zweiten textbasierten Computerdatei, die jede einmalige Textzeichenfolge umfasst, für welche das Verhältnis den Häufigkeitsschwellenwert überschreitet, wobei eine Dateigröße der zweiten textbasierten Computerdatei kleiner als eine Dateigröße der ersten textbasierten Computerdatei ist.

9. Allelotypisierungsverfahren (1000) nach Anspruch 8, wobei die zweite textbasierte Computerdatei eine oder mehrere einmalige Textzeichenfolgen umfasst, die jeweils eine Nukleotidsequenz darstellen, die als Allel für den bestimmten Ort bestimmt ist.

10. Allelotypisierungsverfahren (1000) nach Anspruch 8, wobei die Dateigröße der zweiten textbasierten Computerdatei zumindest zehntausendmal kleiner als die Dateigröße der ersten textbasierten Computerdatei ist.

11. Allelotypisierungsverfahren (1000) nach einem der Ansprüche 1-10, wobei die Schritte des (i) Auswählens (1006) der Vielzahl von Textzeichenfolgen, für welche die dargestellten Nukleotidsequenzen jeweils einem bestimmten Ort entsprechen, (ii) Vergleichens (1010) der ausgewählten Vielzahl von Textzeichenfolgen miteinander, um die Häufigkeitszahl für jede einmalige Textzeichenfolge zu bestimmen, die in der ausgewählten Vielzahl von Textzeichenfolgen beinhaltet ist, und (iii) Bestimmen (1014) von einem oder mehreren Allelen für den bestimmten Ort durch Vergleichen der Häufigkeitszahlen mit dem Häufigkeitsschwellenwert für jeden aus einer Vielzahl von Orten durchgeführt werden, die in einer Probe vorhanden sind, die durch das MPS-Instrument gelesen wurde.

12. Allelotypisierungsverfahren (1000) nach Anspruch 11, wobei das Auswählen der Vielzahl von Textzeichenfolgen, für welche die dargestellten Nukleotidsequenzen jeweils einem aus der Vielzahl von Orten entsprechen, Folgendes umfasst:
Bestimmen (1004), ob jede aus einer Vielzahl von Textzeichenfolgen, die durch das MPS-Instrument beim Lesen der Probe erzeugt werden, Textzeichen beinhaltet, die eine flankierende Nukleotidsequenz darstellen, die einem bestimmten Ort zugeordnet ist; und
Auswählen einer Vielzahl von Textzeichenfolgen, welche die Textzeichen beinhalten, die die flankierende Nukleotidsequenz darstellen, die dem bestimmten Ort zugeordnet ist.

13. Allelotypisierungsverfahren (1000) nach Anspruch 12, ferner umfassend Entfernen der Textzeichen, die die flankierende Nukleotidsequenz darstellen, aus jeder aus der ausgewählten Vielzahl von Textzeichenfolgen vor dem Vergleichen der ausgewählten Vielzahl von Textzeichenfolgen miteinander, um die Häufigkeitszahl für jede einmalige Textzeichenfolge zu bestimmen, die in der ausgewählten Vielzahl von Textzeichenfolgen beinhaltet ist.

14. Allelotypisierungsverfahren (1000) nach einem der Ansprüche 1-13, ferner umfassend Entfernen aller Textzeichen, die keine kurze Tandemwiederholung (STR) darstellen, aus jeder aus der ausgewählten Vielzahl von Textzeichenfolgen vor dem Vergleichen der ausgewählten Vielzahl von Textzeichenfolgen miteinander, um die Häufigkeitszahl für jede einmalige Textzeichenfolge zu bestimmen, die in der ausgewählten Vielzahl von Textzeichenfolgen beinhaltet ist.

## Revendications

1. Procédé d'allélotypage mis en oeuvre par ordinateur (1000) comprenant :
la sélection (1006), parmi un groupe de chaînes de texte qui représentent chacune une séquence de nucléotides qui a été lue par un instrument de séquençage massivement parallèle (MPS), d'une pluralité de chaînes de texte pour lesquelles les séquences de nucléotides représentées par la pluralité sélectionnée de chaînes de texte correspondent chacune à un locus particulier ;
la comparaison (1010) de la pluralité sélectionnée de chaînes de texte les unes aux autres pour déterminer un compte d'abondance pour chaque chaîne de texte unique incluse dans la pluralité sélectionnée de chaînes de texte ; et
la détermination (1014) d'un ou plusieurs allèles pour le locus particulier en comparant le compte d'abondance pour chaque chaîne de texte unique comprise dans la pluralité sélectionnée de chaînes de texte à un seuil d'abondance.

2. Procédé d'allélotypage (1000) selon la revendication 1, ladite détermination (1014) desdits un ou plusieurs allèles pour le locus particulier comprenant l'identification d'une ou plusieurs chaînes de texte uniques qui représentent chacune une séquence de nucléotides contenant une séquence courte répétée en tandem (STR).

3. Procédé d'allélotypage (1000) selon la revendication 1, ladite détermination (1014) desdits un ou plusieurs allèles pour le locus particulier comprenant l'identification d'une ou plusieurs chaînes de texte uniques qui représentent chacune une séquence de nucléotides contenant un polymorphisme nucléotidique simple (SNP).

4. Procédé d'allélotypage (1000) selon l'une quelconque des revendications 1 à 3, ladite comparaison du compte d'abondance pour chaque chaîne de texte unique comprise dans la pluralité sélectionnée de chaînes de texte au seuil d'abondance comprenant l'identification de la chaîne de texte unique comportant le compte d'abondance le plus élevé parmi la pluralité sélectionnée de chaînes de texte.

5. Procédé d'allélotypage (1000) selon la revendication 4, ladite comparaison du compte d'abondance pour chaque chaîne de texte unique comprise dans la pluralité sélectionnée de chaînes de texte au seuil d'abondance comprenant en outre le calcul pour déterminer si le rapport du compte d'abondance pour chaque chaîne de texte unique par rapport au compte d'abondance le plus grand dépasse le seuil d'abondance.

6. Procédé d'allélotypage (1000) selon la revendication 5, ledit seuil d'abondance étant une valeur en pourcentage dans la plage de 15 % à 60 %.

7. Procédé d'allélotypage (1000) selon la revendication 5, ledit seuil d'abondance étant une valeur en pourcentage configurable par un utilisateur.

8. Procédé d'allélotypage (1000) selon l'une quelconque des revendications 5 à 7, comprenant en outre :
la réception (1002) d'un premier fichier informatique basé sur du texte comprenant un groupe de chaînes de texte qui représentent chacune une séquence de nucléotides qui a été lue par l'instrument MPS, avant la sélection de la pluralité de chaînes de texte pour lesquelles les séquences de nucléotides représentées correspondent chacune au locus particulier ; et
la génération d'un second fichier informatique à base de texte comprenant chaque chaîne de texte unique pour laquelle le rapport dépasse le seuil d'abondance, la taille de fichier du second fichier informatique à base de texte étant inférieure à la taille de fichier du premier fichier informatique à base de texte.

9. Procédé d'allélotypage (1000) selon la revendication 8, ledit second fichier informatique à base de texte comprenant une ou plusieurs chaînes de texte uniques qui représentent chacune une séquence de nucléotides déterminée comme étant un allèle pour le locus particulier.

10. Procédé d'allélotypage (1000) selon la revendication 8, ladite taille du second fichier informatique à base de texte étant au moins dix mille fois inférieure à la taille de fichier du premier fichier informatique à base de texte.

11. Procédé d'allélotypage (1000) selon l'une quelconque des revendications 1 à 10, lesdites étapes de (i) sélection (1006) de la pluralité de chaînes de texte pour lesquelles les séquences de nucléotides représentées correspondent chacune à un locus particulier, (ii) comparaison (1010) de la pluralité sélectionnée de chaînes de texte les unes aux autres pour déterminer le compte d'abondance pour chaque chaîne de texte unique comprise dans la pluralité sélectionnée de chaînes de texte, et (iii) détermination (1014) d'un ou plusieurs allèles pour le locus particulier en comparant les comptes d'abondance au seuil d'abondance étant effectuées pour chacun d'une pluralité de locus présents dans un échantillon qui a été lu par l'instrument MPS.

12. Procédé d'allélotypage (1000) selon la revendication 11, ladite sélection de la pluralité de chaînes de texte pour lesquelles les séquences de nucléotides représentées correspondent chacune à l'un de la pluralité de locus comprenant :
la détermination (1004) pour savoir si chacune d'une pluralité de chaînes de texte générées par l'instrument MPS lors de la lecture de l'échantillon comprend des caractères de texte qui représentent une séquence de nucléotides adjacente associée à un locus particulier ; et
la sélection d'une pluralité de chaînes de texte qui comprennent les caractères de texte qui représentent la séquence de nucléotides adjacente associée au locus particulier.

13. Procédé d'allélotypage (1000) selon la revendication 12, comprenant en outre la suppression des caractères de texte qui représentent la séquence de nucléotides adjacente de chacune de la pluralité sélectionnée de chaînes de texte avant la comparaison de la pluralité sélectionnée de chaînes de texte les unes aux autres pour déterminer le compte d'abondance pour chaque chaîne de texte unique comprise dans la pluralité sélectionnée de chaînes de texte.

14. Procédé d'allélotypage (1000) selon l'une quelconque des revendications 1 à 13, comprenant en outre la suppression de tous les caractères de texte qui ne représentent pas une séquence courte répétée en tandem (STR) de chacune de la pluralité sélectionnée de chaînes de texte avant la comparaison de la pluralité sélectionnée de chaînes de texte les unes aux autres pour déterminer le compte d'abondance pour chaque chaîne de texte unique incluse dans la pluralité sélectionnée de chaînes de texte.
